(19) **Europäisches Patentamt European Patent Office Office européen des brevets**

(11) **EP 1 893 661 B1**

(12) **EUROPEAN PATENT SPECIFICATION**

(45) Date of publication and mention of the grant of the patent:
**18.01.2012 Bulletin 2012/03**

(51) Int Cl.:
*C08G 4/00* *(2006.01)* *A61K 47/48* *(2006.01)*

(21) Application number: **06770201.9**

(22) Date of filing: **10.05.2006**

(86) International application number:
**PCT/US2006/018182**

(87) International publication number:
**WO 2006/122223 (16.11.2006 Gazette 2006/46)**

(54) **STRATEGIES FOR DELIVERY OF ACTIVE AGENTS USING MICELLES AND PARTICLES**

STRATEGIEN ZUR FREISETZUNG VON WIRKSTOFFEN UNTER VERWENDUNG VON MIZELLEN UND PARTIKELN

NOUVELLES STRATEGIES D'ADMINISTRATION D'AGENTS ACTIFS UTILISANT DES MICELLES ET DES PARTICULES

(84) Designated Contracting States:
**AT BE BG CH CY CZ DE DK EE ES FI FR GB GR HU IE IS IT LI LT LU LV MC NL PL PT RO SE SI SK TR**
Designated Extension States:
**AL BA HR MK YU**

(30) Priority: **10.05.2005 US 679480 P**
**23.09.2005 US 720099 P**

(43) Date of publication of application:
**05.03.2008 Bulletin 2008/10**

(73) Proprietors:
• **Emory University**
**Atlanta, GA 30322 (US)**
• **GEORGIA TECH RESEARCH CORPORATION**
**Atlanta, GA 30332-0415 (US)**
• **University of Rochester Medical Center**
**Rochester, NY 14642 (US)**

(72) Inventors:
• **PULENDRAN, Bali**
**Alpharetta, GA 30022 (US)**
• **MURTHY, Niren**
**Atlanta, GA 30308 (US)**
• **PIERCE, Robert H.,**
**Palo Alto, California 94306 (US)**

• **HEFFERNAN, Michael, J.**
**Atlanta, GA 30350 (US)**
• **HAO, Jihua**
**Beachwood, OH 44122 (US)**
• **KWISSA, Marcin,**
**EMORY UNIVERSITY**
**Atlanta, GA 30329 (US)**

(74) Representative: **Kalhammer, Georg et al**
**Lederer & Keller**
**Patentanwälte**
**Unsöldstrasse 2**
**80538 München (DE)**

(56) References cited:
**WO-A2-2005/023294**

• **ST. PIERRE, THOMAS ET AL: "Biodegradability of synthetic polymers for medical and pharmaceutical applications: Part 2 - Backbone hydrolysis" JOURNAL OF BIOACTIVE AND COMPATIBLE POLYMERS , 2(1), 1-30 CODEN: JBCPEV; ISSN: 0883-9115, 1987, XP009073664**

Remarks:
The file contains technical information submitted after the application was filed and not included in this specification

**Description**

**FIELD OF THE INVENTION**

The invention relates to particle and micelle based strategies for delivering active agents, such as (i) vaccines; (ii) immune modulatory agents, (including TLR ligands or synthetic molecules, which modulate the function of innate immune cells such as dendritic cells, or synthetic molecules or siRNA that modulate signaling networks within cells (e.g., dendritic or other antigen presenting cells) and/or; (iii) drugs that target antigen-presenting cells so as to modulate innate and adaptive immunity, in a therapeutic or prophylactic setting.

**BACKGROUND OF THE INVENTION**

*Harnessing innate immunity for vaccination*

A hallmark of the immune system is its ability to launch qualitatively different types of immune responses. Thus for example, T-helper 1 (or Th1 immune responses stimulate cytotoxic "killer" T cells, which kill virally infected cells or tumors. In contrast, T-helper 2 (or Th2) responses are associated with antibody production, particularly secretion of IgE antibodies, which confer protection against extracellular parasites or bacteria or toxins. Furthermore, T regulatory responses can suppress over exuberant immune responses, and thus limit the immune pathology caused by allergies, autoimmunity, transplant rejection, or sepsis like symptoms. Given the existence of such diverse types of immune responses, and their differential roles in conferring effective protection against viruses, tumors, extracellular parasites and bacteria, and in regulating deleterious immune responses in allergies, autoimmunity, transplantation and sepsis, a "rosetta stone" of modern immunology is to learn how to induce optimally effective immune responses in various clinical settings.

In this context, recent advances in immunology have revealed a fundamental role for the innate immune system in controlling both the quality and quantity of immune responses (Pulendran & Ahmed, Cell, 2006, 124:849-863). Thus, it has long been known that the immune system is unresponsive to most foreign proteins that are injected in a soluble, deaggregated form, but when injected together with immune-stimulating substances called "adjuvants," these foreign proteins can induce robust immunity. In fact it was known that the nature of the adjuvant is what determines the particular type of immune response that follows, which may be biased towards cytotoxic T-cell responses, antibody responses, or particular classes of T-helper responses (Pulendran, Immunol. Rev., 2004, 199:227-250; Pulendran, J. Immunol., 2005, 175:2457-2465; Pulendran & Ahmed, Cell, 2006, 124:849-863). Despite the importance of adjuvants, there is only one adjuvant, alum, licensed for clinical use in the United States, and most other experimental adjuvants consist of crude extracts of microbes or bacteria, which induce potent activation of immune cells, but also result in toxicities. Until recently, the mechanism of action of such adjuvants was not understood. However, recent advances in innate immunity have offered a conceptual framework with which to understand how adjuvants function. Central to this issue is a rare but widely distributed network of cells known as dendritic cells (DCs), which constitute an integral component of the innate immune system. DCs, which have been called 'Nature's adjuvants,' express receptors which can recognize components of microbes and viruses. Such receptors include the Toll-like receptors (TLRs), C-type lectins, and CATERPILLAR proteins, which can "sense" microbial stimuli, and activate DCs and other immune cells (Pulendran, Immunol. Rev., 2004, 199:227-250; Pulendran, J. Immunol., 2005, 175:2457-2465; Pulendran & Ahmed, Cell, 2006, 124:849-863]. It is now clear that DCs play essential roles in orchestrating the quality and quantity of the immune response.

There are currently some 13 TLRs described in mammals. Activating distinct TLRs on DCs induces qualitatively different types of immune responses (Pulendran, et al, 2001, *supra;* Dillon et al, 2004, *supra;* Agrawal et al, J. Immunol., 2003, 171:4984-4989; Dillon et al, J. Clin. Immunol., 2006, 116:916-928). Thus, activating most TLRs can induce Th1 responses; activating TLR3, 7 or 9 can induce cytotoxic T cells that kill virally infected cells and tumors; and emerging evidence suggests that activating TLR2 induces Th2 responses, (which are associated with antibody responses that offer protection against viruses or extracellular bacteria or parasites), or even T regulatory or tolerogenic responses, (which suppress over exuberant immune responses, and thus offer protection against unbridled immunity in allergies, autoimmunity, sepsis, and transplantation). As such, DCs and TLRs and other recognition receptors; represent attractive immune modulatory targets for vaccinologists and drug developers. Thus learning how to exploit fundamental elements of the innate immune systems such as DCs and TLRs, is of paramount importance in the development of novel drugs and vaccines.

An important corollary to this notion is that the vast majority of vaccines which have been developed over the past 200 years, (since the first recorded vaccination trial of Edward Jenner), have been developed empirically. Therefore, despite their successes in controlling various scourges such as smallpox, polio, TB and yellow fever, we have no knowledge of the scientific rationale for how these vaccines stimulate such effective immunity. For example, the yellow fever vaccine 17D [YF-17D] is one of the most effective vaccines known. Since its development more than 65 years ago, it has been administered to over 400 million people globally. Despite its success, the mechanism of its action is not known. Therefore, as stated above, the spectacular advances in innate immunity which have occurred in the last six years or so, offer us a new vision with which to understand the modus operandi of such "gold standard" vaccines, with a view to using such knowledge to devising future vaccines against emerging and re-emerging infections of the 21st century. In this context, our recent findings suggest that the highly effective Yellow Fever Vaccine (YF-17D) is a potent stimulator of DCs, and multiple TLRs, including TLR 2, 7, 8 and 9 (Querec et al., J. Exp. Med., 2006, 203:413-421). Given, the different types of immune responses triggered by the distinct TLRs (Pulendran et al., 2001, *supra;* Agrawal et al., J. Immunol., 2003, 171:4984-4989; Dillon et al, 2004, *supra;* Dillon et al, J. clin. Immunol., 2006, 116:916-928), it was tempting to speculate that by activating multiple TLRs, YF-17D was inducing a broad spectrum of immune responses. Indeed, our data suggests that YF-17D triggers a broad spectrum of innate and adaptive immune responses (Th1 Th2, cytotoxic T cells, neutralizing antibody), and that distinct TLRs control different types of this polyvalent immunity (Querec et al., J. Exp. Med., 2006, 203:413-421). Eliciting such a broad spectrum of immune responses is also likely to be beneficial in designing vaccines against other infections, against which no effective vaccines currently exist, such as HIV, HCV, malaria, TB, influenza, anthrax and Ebola, or against tumors. Thus, strategies for designing future vaccines against emerging or re-emerging infections might benefit from incorporating multiple TLR ligands plus antigens, plus immune modulatory agents, in order to induce multi-pronged immune responses. Therefore, an important challenge is the development of delivery systems which are capable of delivering such immune modulatory agents in vivo.

### Delivery systems for novel vaccines

Drug delivery vehicles based on polyesters and polyanhydrides have been widely used for the sustained release of therapeutics because of their excellent biocompatibility profiles and slow hydrolysis rates (Anderson, J.M. et al., Adv. Drug Delivery Rev., 1997, 28:5-24; Jain, R.A., Biomaterials, 2000, 21:2475-2490; Mathiowitz, E. et al., J. Appl. Polym. Sci., 1988, 35:755-774; Berkland, C. et al., J. Controlled Release, 2004, 94:129-141). However, numerous medical applications, such as targeting the acidic environment of lysosomes and tumors, require drug delivery systems that undergo rapid, pH-sensitive degradation (Stubbs, M. et al., Mol. Med. Today, 2000, 6:15-19; Leroux, J.-C., Adv. Drug Delivery Rev., 2004, 56:925-926). The majority of degradable polymers used for drug delivery cannot fulfill this requirement because they are composed of ester linkages, which degrade by base-catalyzed hydrolysis at physiological pH values. Particles made of ester based materials, such as Poly(lactic-glycolic acid) (PLGA), polyorthoesters, and polyanhydrides, all generate high quantities of acid when they degrade. This causes degradation of protein and DNA therapeutics and the degradation also takes weeks to months. Because the life span of mature DCs is around 2 days these materials are not ideal for vaccine development. Recently, pH sensitive hydrophobic microparticles based on poly(orthoesters) and poly(beta-amino esters) have been successfully used for intracellular drug delivery and tumor targeting, thus demonstrating the potential of acid-sensitive biomaterials for drug delivery (Heller, J. et al., Biomacromolecules, 2004, 5:1625-1632; Heller, J. et al., Adv Drug Delivery Rev., 2002, 54:1015-1039; Berry, D. et al., Chem. Biol., 2004, 11:487-498; Potineni, A. et al., J. Controlled Release, 2003, 86:223-234). Consequently, there is great interest in developing new strategies for the synthesis of pH-sensitive biodegradable polymers.

Vaccines based on recombinant proteins, peptide antigens, or DNA vaccines encoding such vaccine antigens, have tremendous therapeutic potential against infectious diseases and tumors, in which the antigenic epitopes have been defined. Such vaccines have been capable of generating protective immunity against infectious diseases, in animal models, and numerous clinical trials with such vaccines are currently in progress (van Endert, PM, Biologicals, 2001, 29:285-8; Purcell, AW et al., Journal of Peptide Science, 2003, 9:255-81; Shirai, M. et al., Journal of Virology, 1994, 68: 3334-42; Hunziker, IP et al., International Immunology, 2002, 14:615-26). However, despite their promise, a major challenge concerns the efficient delivery of peptides, proteins, DNA vaccines and adjuvants, so as to target the appropriate type of antigen presenting cell in order to launch an effective immune response. Although promising results have been obtained with peptide vaccines composed of lipid conjugates and PLGA microparticles, there is still a great need for the development of new peptide vaccine delivery vehicles (Ertl, HCJ et al., Vaccine, 1996, 14:879-85; Jackson, DC et al., Vaccine, 1997, 15:1697-705).

## SUMMARY OF THE INVENTION

The present invention provides biodegradable particles (e.g., three-dimensional particles) and micelles which can be used to encapsulate active agents for delivering to a subject. The present invention further provides methods for producing and delivering such particles and micelles. Additionally, the invention provides vaccination strategies that encompass the use of the novel particles and micelles.

*Hydrophobic polyketal particles*

The present invention is directed to new type of hydrophobic polymers comprising ketal groups in the polymer backbone wherein the ketal groups are arranged in a way such that both oxygen atoms are located in the polymer backbone.

Further, the ketal polymer can be formed via a ketal exchange reaction between a ketal and a diol. In accordance with the invention, one or more types of the ketals and/or diols can be used for the formation of a homopolymer or copolymer.

Also encompassed by the present invention are polyketal polymers which are joined by other polymers (e.g. PEG, polyesters, polyamides, polysaccharides, polyethers, or polyanhydrides). The resulting polymers can be alternating copolymers, random copolymers, block copolymers, or graft copolymers. Polythioketal polymers, mixed polythio-amine ketals, polythio-hydroxyl ketals and polyhydroxyl-amine ketals are also in the scope of the present invention.

Polyketal polymers of the invention hydrolyze in aqueous solutions into low molecular weight, water soluble alcohols and ketones. The advantage of a ketal linkage in the backbone is that it degrades under acidic conditions of phagosomes, within 1-2 days at pH 5.0. Polyketals can therefore also be used for targeting the acidic environments of tumors, inflammation and phago-lysosomes. The degradation does not generate acidic degradation products. Thus, the ketal polymers are suitable for biological use.

*Micelles*

The present invention further provides novel biodegradable crosslinked micelles comprising multiple polymers, wherein the polymers are e.g.,. crosslinked by an external crosslinking agent (i.e., agents which are not introduced into the polymer chain). The advantage of using of external agents is the faster crosslinking reaction compared to a reaction wherein only crosslinkable moieties within the polymer are used. The external crosslinking agent also decreases the chances that the encapsulated is protein destroyed.

**BRIEF DESCRIPTION OF THE FIGURES.**

Figure 1 is a bar graph showing the fluorescence intensity of filtered and unfiltered PKNs with FITC-Ova (excitation 494 nm, emission 520 nm).

Figure 2 is a diagrammatic representation showing the synthesis and degradation of Ketal-backbone polymer (polyketal).

Figure 3(A) is a graph showing a GPC trace of polyketal 2 (of Fig. 2) in THF and Figure 3(B) is a $^1$H NMR spectrum of polyketal 2 (of Fig. 2) in CDCl$_3$.

Figure 4 is a graph showing the hydrolysis kinetics of polyketal 2 (of Fig. 2).

Figure 5 shows SEM images of particles made with polyketal 2 (of Fig. 2).

Figure 6 shows a schematic representation of particle formation.

Figure 7 is a photograph showing a polyketal particles loaded with Fluorescein are taken up in the liver.

Figure 8 is a schematic diagram showing a peptide crosslinked micelle design and synthesis.

Figure 9 shows the synthesis and characterization of PEG-polylysine thiopyridal. A. is a chemical diagram showing the synthesis of PEG-polylysine thiopyridal. B is a 1H-NMR spectrum of PEG-PLL-thiopyridal in D$_2$O. C./D are graphs depicting the dynamic light scattering analysis of PCMs uncross-linked (C) and peptide cross-linked (D). E is a graph showing the crosslinking reaction of cysteines on peptide anigen II (Fig. 8). F is a graph showing the UV analysis of crosslinking reaction between peptide anigen II (Fig. 8) and block copolymer micelles.

Figure 10 shows the effect of GSH on release of peptides and DNA A is a graph showing GSH sensitive peptide release. B is a gel elecrophoresis analysis showing GSH sensitive DNA release. C is a gel elecrophoresis analysis showing ISS-DNA is protected from serum nucleases in the PCMs.

Figure 11 depicts block copolymer micelles. A depicts the chemical structure of PEG-poly(lysine-thio-pyridyl). B is a schematic diagram showing micelle formation. C is a schematic diagram showing crosslinking of micelles. D is a schematic diagram showing reducing of crosslinked micelles.

Figure 12 shows the immunology of micelle. A. Confocal microscopic analysis of the uptake of SIINFEKL-CFSE micelles by human monocyte derived DCs. B. FACS analysis of uptake of micelle encapsulated SIINFEKL peptide by human monocyte derived DCs. C. FACS analysis of uptake of micelle encapsulated SIINFEKL peptide by mouse DCs and Macrophages.

Figure 13 is a bar graph showing micelle formulated SIINFEKL peptide induces potent T cell responses *in-vitro.*

Figure 14 shows the immunology of micelle. A shows FACS analysis of efficient uptake of micelle encapsulated OVA protein by mouse DCs and Macrophages. B is a graph showing that micelle encapsulated OVA/CpG activates DCs *in-vitro.*

Figure 15 is a graph depicting the immunology of polyketal particles. Uptake of polyketal particle (PKN) encapsulated U0126 by mouse DCs and Macrophages *in-vitro.*

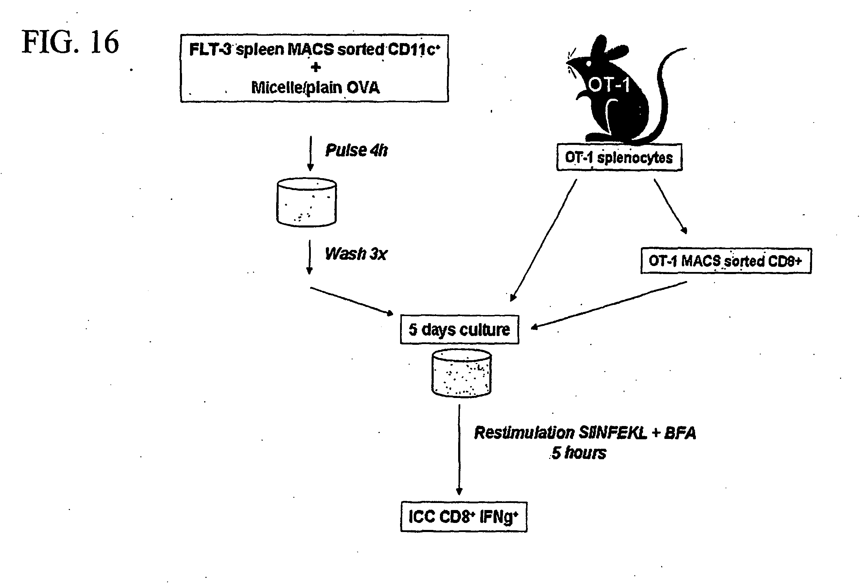

Figure 16 is a schematic diagram showing the experimental outline for T cell stimulation in-vitro using OVA-OT/1 transgenic model.

Figure 17 shows the immunology of micelle. A. Left panel is the flow cytometery analysis and the right panel is the summary showing that splenocytes pulsed with micelle formulated antigen induces potent antigen-specific CD8+ T cell responses *in-vitro. B.* Left panel is the flow cytometery analysis and the right panel is the summary showing that DCs pulsed with micelle formulated antigens induce potent antigen-specific CD8+ T cell responses in vitro. C. Flow cytometery analysis showing that micelle formulated antigen activate DCs *in-vivo.*

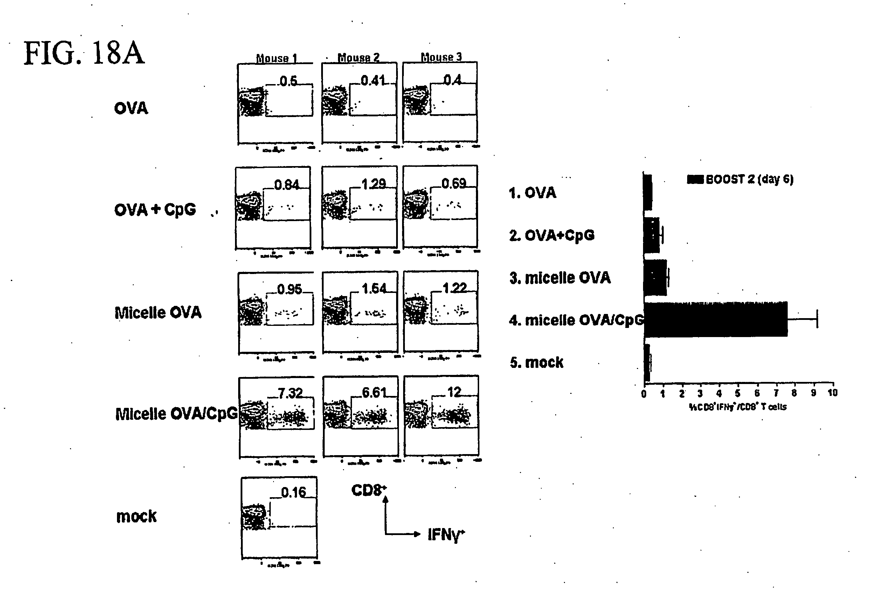

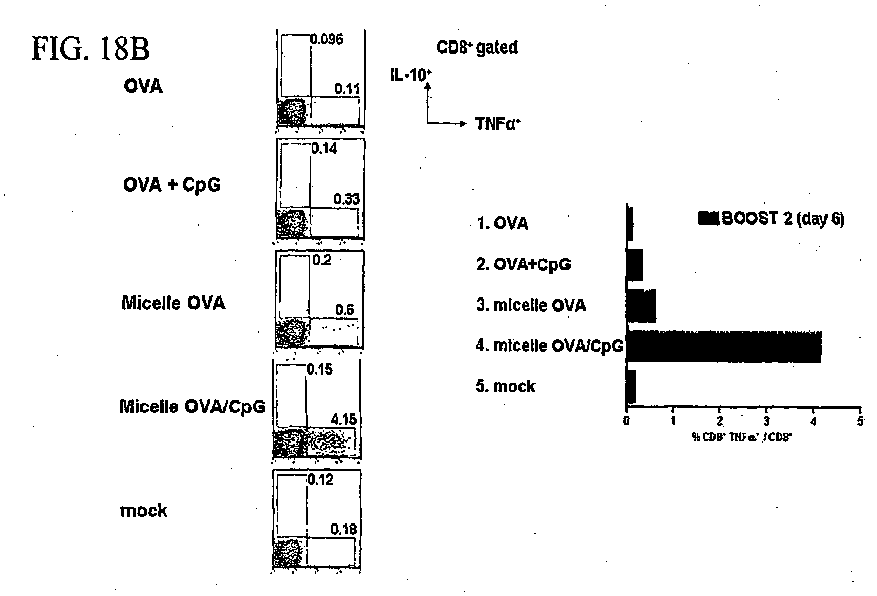

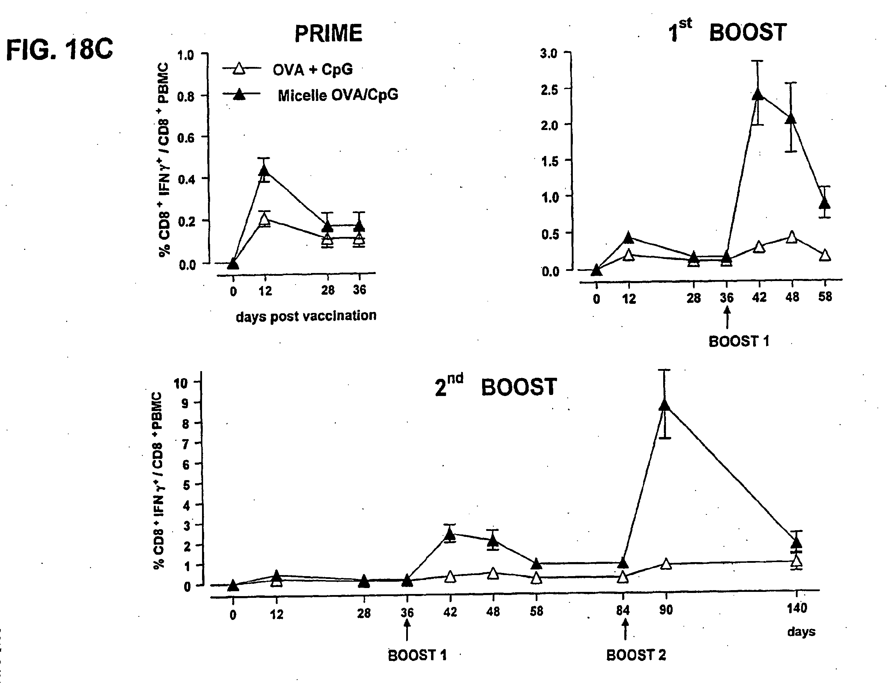

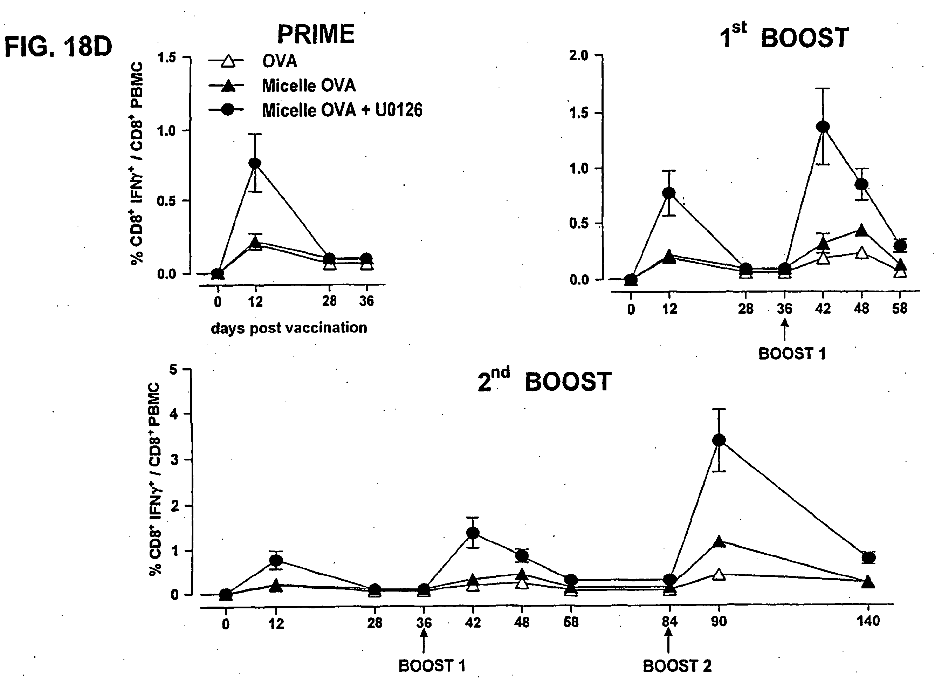

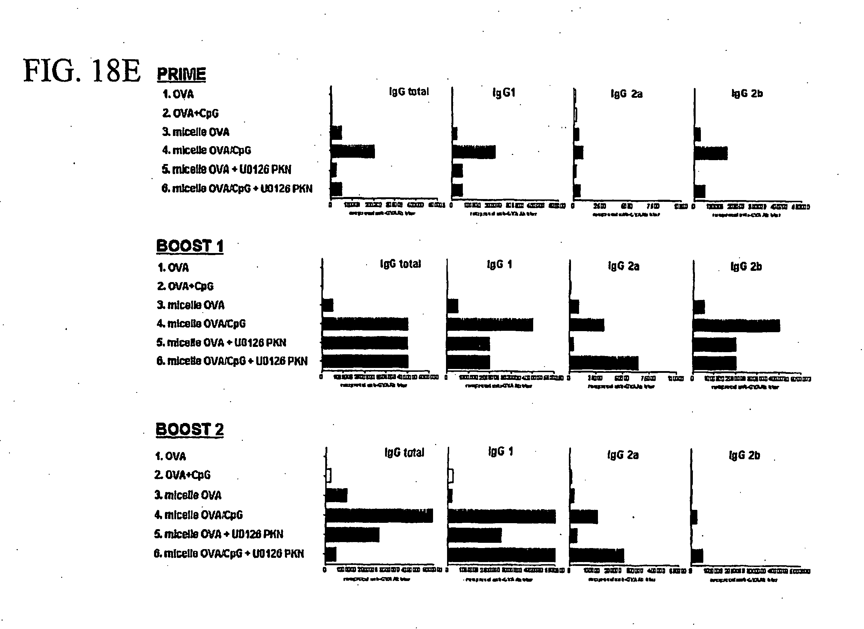

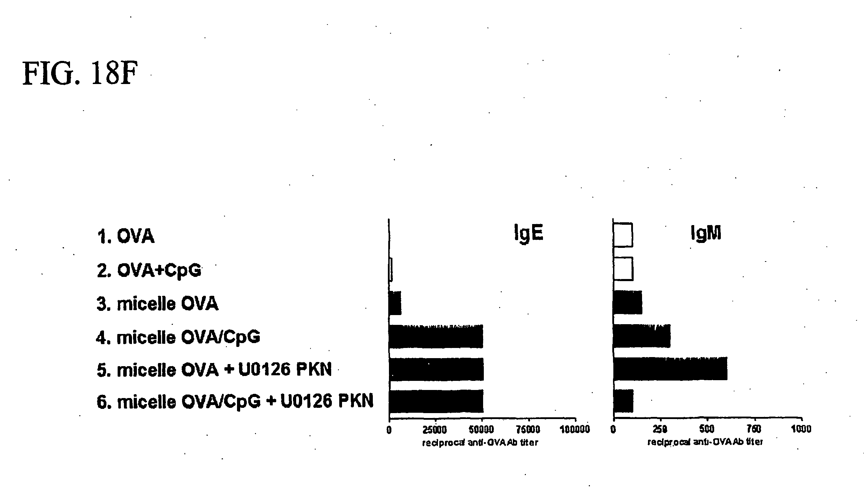

Figure 18 shows the immunology of micelle and polyketal particles. A. Left panel is the flow cytometery analysis and the right panel is the summary showing that micelle formulated vaccines induce strong antigen-specific CD8+ IFN-gamma+ T cell responses *in-vivo .* B. Left panel is the flow cytometery analysis and the right panel is the summary showing that micelle formulated vaccines induce strong antigen-specific $CD8^+$ $TNF\alpha^+$ T cell responses *in-vivo.* C. Line graphs showing the kinetics of specific $CD8^+/IFN\gamma^+$ T cells after OVA/CpG vaccination. D. Line graphs showing the kinetics of specific $CDS^+/IFN\gamma^+$ T cells after OVA + UO126 PKN vaccination. E. Bar graphs showing that micelle formulated vaccines induce strong antigen specific IgG antibody response *in-vivo.* F. Bar graphs showing that micelle formulated vaccines induce antigen specific IgE and IgM antibody response *in-vivo..*

Figure 19 shows polyketals from cyclohexane dimethanol (termed PCADK). A. Chemical representation showing polyketals from cyclohexane dimethanol. B. Line graph showing that PCADK degrades in an acid sensitive manner.

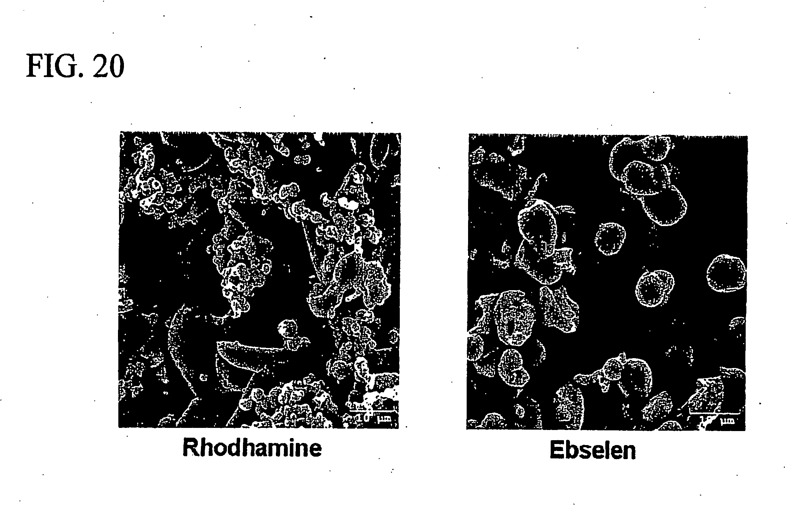

Figure 20 shows SEM images depicting that particles from PCADK can encapsulate the hydrophobic compounds and drugs such as rhodhamine red and ebselen.

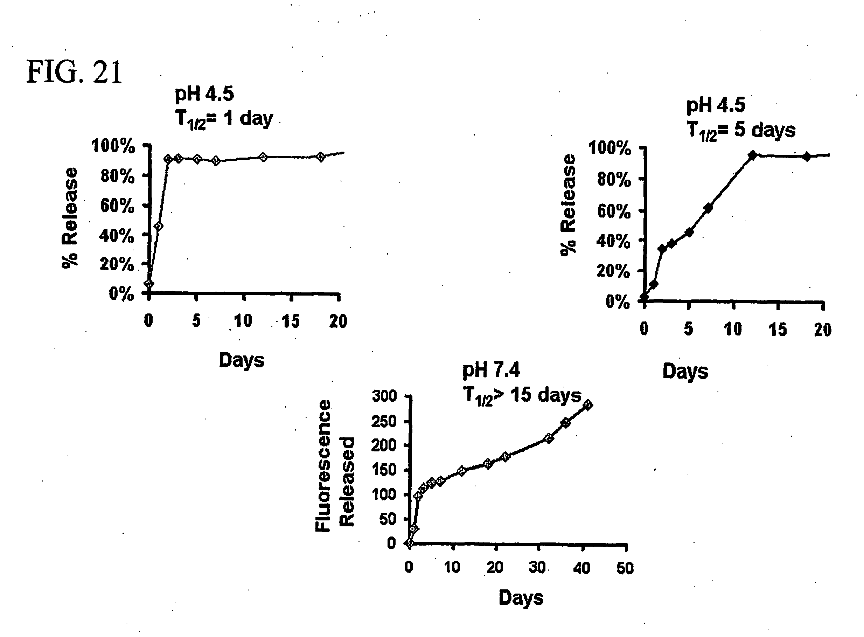

Figure 21 shows line graphs showing that release of rhodhamine red from PCADK is pH sensitive.

Figure 22 is a chemical representation showing that polyketals with almost any aliphatic diol can be made.

Figure 23 is a photograph showing that FITC labeled polyketals are phagocytosed by liver macrophages.

Figure 24A is a schematic diagram showing double emulsion procedure used to encapsulate catalase and super oxide dismutase in polyketal particles. B is a SEM image of catalase containing particles, and fluorescent microscope images of catalase containing particles. C is a graph showing that catalase particles have enzymatic activity.

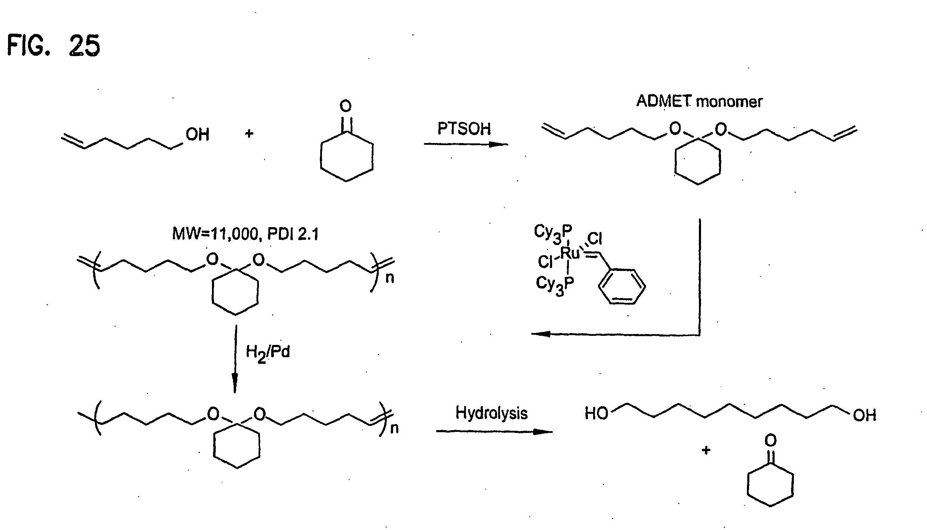

Figure 25 is a chemical representation showing that polyketals made by acyclic diene metathesis (ADMET).

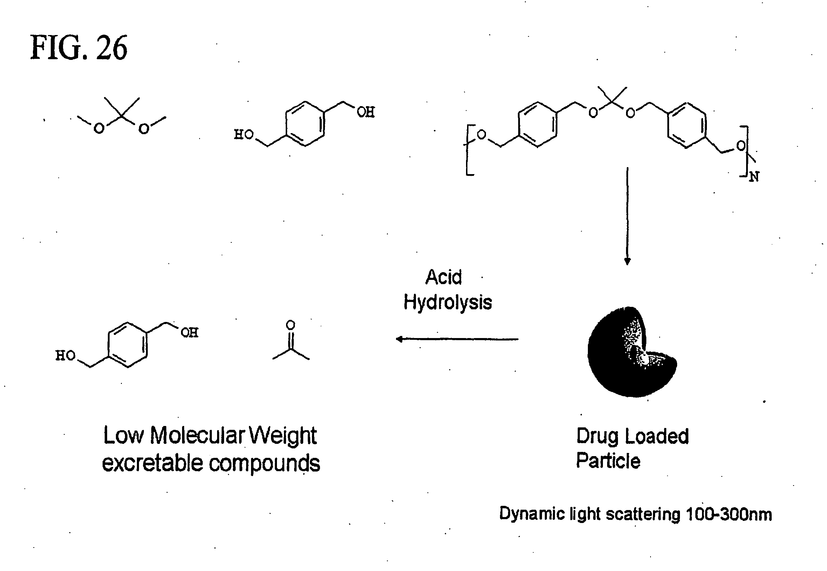

Figure 26 is a schematic diagram for the synthesis and acid degradation of drug loaded particles.

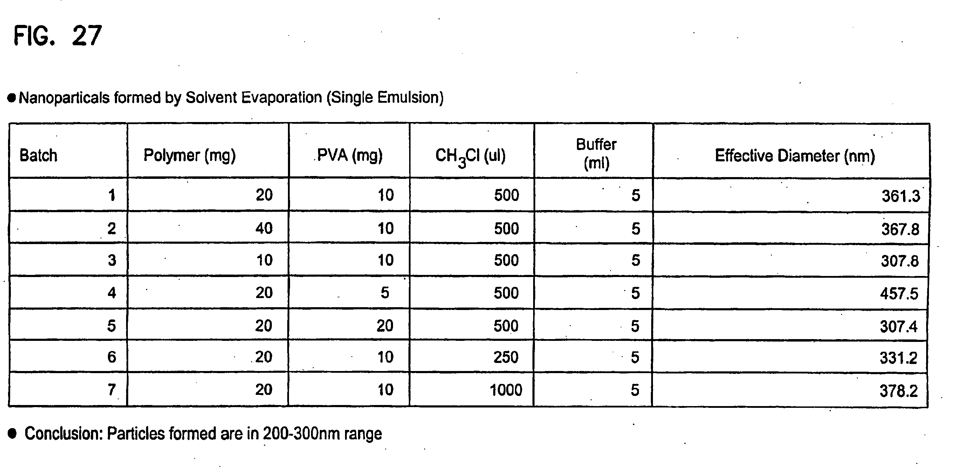

Figure 27 is a table showing the conditions used to make different rhodhamine containing PCADK particles.

## DETAILED DESCRIPTION OF THE INVENTION

### DEFINITIONS

All scientific and technical terms used in this application have meanings commonly used in the art unless otherwise specified. As used in this application, the following words or phrases have the meanings specified.

As used herein, the term "micelle" refers to a colloidal aggregate of polymer molecules having at least two different moieties which are linked to different properties in a liquid medium. The difference in the properties can occur due to different hydrophobicity/hydrophilicity, polarity, charge or charge distribution or other parameters which influence the solubility of a molecule. Micelles of the present invention are distinguished from and exclude liposomes which are composed of bilayers.

As used herein, the term "polymer" refers to a covalently linked arrangement of monomeric molecules. The arrangement can be realized in a linear chain or in a branched form. The polymer can be a homopolymer which is composed of only one type of monomeric molecules, or it can be a copolymer wherein two or more different types of monomers are joined in the same polymer chain. When the two different monomers are arranged in an alternating fashion, the polymer is called an alternating copolymer. In a random copolymer, the two different monomers may be arranged in any order. In a block copolymer each type of monomer is grouped together. A block copolymer can be thought of as two or more homopolymers joined together at the ends. When chains of a polymer made of one monomer are grafted onto a polymer chain of a second monomer a graft copolymer is formed.

As used herein, the term "particle" or "three-dimensional particle" refers to nanoparticles and/or microparticles. According to standard definitions, the term "nanoparticle" covers only particle having at least one dimension smaller than 100 nm. Larger particle which do not fulfill this requirement are termed "microparticles". The present invention provides three-dimensional particles sized on the nanometer (nm) and micron ($\mu$m) scale.

The terms "ketals" and "diols" as used herein encompass ketals and diols comprising alkyl, cycloalkyl and aryl groups. "alkyl group" or "aliphatic group" as used herein, is linear or branched chain alkyl group. In one embodiment a linear alkyl group is preferred. Also included within the definition of alkyl are heteroalkyl groups, wherein the heteroatom can be nitrogen, oxygen, phosphorus, sulfur and silicon.

The term "cycloalkyl group" or "cycloaliphatic group", as used herein describes a ring-structured alkyl including at least three carbon atoms in the ring. In one embodiment, a cycloalkyl group having 5 or 6 carbons is preferred. The cycloalkyl group also includes a heterocyclic ring, wherein the heteroatom can be nitrogen, oxygen, phosphorus, sulfur and silicon.

"Aryl group" or "aromatic group" as used herein, is an aromatic aryl ring such as phenyl, heterocyclic aromatic rings such as pyridine, furan, thiophene, pyrrole, insole and purine, and heterocyclic rings with nitrogen, oxygen, sulfur or phosphorus. Included in the definition of alkyl, cycloalkyl and aryl groups are substituted alkyl, cycloalkyl and aryl groups. These groups can carry one or more substitutions. Suitable substitution groups include but are not limited to, halogens, amines, hydroxyl groups, carboxylic acids, nitro groups, carbonyl and other alkyl, cycloalkyl and aryl groups.

In order that the invention herein described may be more fully understood, the following description is set forth.

### COMPOSITIONS OF THE INVENTION

Polyketal polymers of the invention

The present invention provides biodegradable hydrophobic polyketal polymers comprising multiple ketal groups, each ketal group having two oxygen atoms within the polymer backbone.

Examples of suitable ketal groups include, but are not limited to, 2,2-dioxypropyl group, 2,2-dioxybutyl group, 1,1-dioxycyclohexyl group or dioxyacetophenyl group. Also in the scope of the invention are ketal polymers including aliphatic, cycloaliphatic or aromatic ketals containing one or more hetero-atom, such as nitrogen, sulfur, oxygen and halides.

In one embodiment of the invention, the polymer further comprises a compound comprising alkyl, aryl, and cycloalkyl groups. In this embodiment, the compound may be directly attached to the ketal group.

Examples of suitable alkyl groups include, but are not limited to, methyl, ethyl and butyl groups. Examples of suitable aryl groups include, but are not limited to, substituted or unsubstituted benzyl, phenyl or naphtyl groups, such as, for example, a 1,4-dimethylbenzene. Examples of suitable cycloalkyl groups include, but are not limited to, substituted or unsubstituted cyclohexyl, cyclopropyl, cyclopentyl groups, such as, for example, 1,4-dimethylcyclohexyl group.

In a preferred embodiment, the polymer may be poly(1,4-phenylene-acetone dimethylene ketal). This polymer can be synthesized of 2,2-dimethoxypropane and 1,4-benzene dimethanol. The polymer may also be a poly(1,4-cyclohexane-acetone dimethylene ketal), which can be synthesized of 2,2-dimethoxypropane and 1,4-cyclohexane dimethanol.

The invention further provides biodegradable particles comprising the polyketal polymers of the invention. The sizes of the particles can vary. For example, biodegradable particles can be made at nanometer (nm) or micron (μm) scale. The preferred particle size is between about 50 and 1000 nm, more preferred between about 200 and 600 nm.

The preferred size of a suitable polyketal polymer to form the biodegradable particle is between about 0.5 kDa and about 2 MDa, more preferred between 1 and about 150 kDa, most preferred between about 4 and about 6 kDa. In accordance with the invention, the number of the monomers in the polymer can range from about 2 to about 20,000, preferably about 10 to about 1,000, more preferably about 10 to about 50.

Polyketal polymers of the invention hydrolyze in aqueous solutions into low molecular weight, water soluble alcohols and ketones. For example, the degradation of poly(alkyl-acetone dimethylene ketal) is acid sensitive, with a half-life of 102.0 h at pH 7.4 and 35.0 h at pH 5.0. The advantage of a ketal linkage in the backbone is that it degrades under acidic conditions of phagosomes, within 1-2 days at pH 5.0. Polyketals can therefore also be used for targeting the acidic environments of tumors, inflammation and phago-lysosomes. The degradation also does not generate acidic degradation products. Thus, the ketal polymers are suitable for biological use.

In accordance with the practice of the invention, the polyketal polymer, particles can further comprise one or more active agents. As used herein, the term "active agent" refers to a protein, peptide, nucleic acid (DNA or RNA) or organic molecule, and other synthetic nucleic acid molecules, siRNA molecules, or antisense molecules. The active agent can be an immunomodulatory agent such as specific ligands for RIG-1 or TLRs, or C-type lectins (such as dectin-1 and DC-SIGN) or caterpillar proteins, or combinations of specific TLR ligands, or synthetic molecules or siRNAs which inhibit regulatory signaling networks with DCs and macrophages. The active agent can further include recombinant proteins, vaccine antigens, DNA vaccines, or vaccines themselves, such as the influenza vaccine. Finally, an active agent can comprise antibodies that target, stimulate, modulate or inhibit subsets of DCs including Langerhans cells, dermal DCs, myeloid DCs, interstitial DCs, plasmacytoid DCs, or subsets of monocytes, and macrophages. Thus surface of these particles can be modified to contain targeting groups such as, for example, antibodies against subsets of dendritic cells, or proteins which stimulate subsets of dendritic cells, or macrophages such as CD40L, DEC-205, CD11c, langerin, MARCO, 33D1 etc.

Examples of suitable active agents include, but are not limited to: (1) agonists and antagonists of TLRs (*e.g.*, TLR-2, TLR-3, TLR-4, TLR-5, TLR-7, TLR-8, TLR-9, TLR-10, and TLR-11), (2) agonists and antagonists of the receptor(s) activated by schistosome egg antigen (SEA), (3) molecules that stimulate or inhibit the expression or activity of a component of an intracellular signaling pathway that transduces the signal generated by activation of either of these types of receptors, and (4) agents that stimulate or inhibit a transcription factor that is induced or stabilized by one or more of these signaling pathways.

Examples of agonists include, but are not limited to, peptidoglycans (O. Takeuchi, et al., 1999 Immunity 11: 443-451) or zymosans (Dillon et al, 2006 J. Clin. Invest. 116(4):916-28). A. Ozinsky, et al., 2000 Proc. Natl. Acad. Sci. USA 97:13766-13771). The agonists also include bacterial lipopeptides (e.g., diacylated and triacylated lipopeptides), lipoteichoic acid, lipoarabinomannan, phenol-soluble modulin, glycoinositolphospholipdis, glycolipids, porins, atypical LPS from Leptospira interrogns or Porphyromonas gingivalis, or HSP70 (for a review see K Takeda, et al., 2003 Annu. Rev. Immunol. 21:335-376). The agonists can be isolated and/or highly purified molecules. The agonists include whole molecules or fragments thereof, or naturally-occurring or synthetic. Examples include, but are not limited to, a non-toxic form of cholera toxin (Braun et al., J. Exp. Med 189:541-552, 1999), certain forms of *Candida albicans* (d'Ostiani et al., J. Exp. Med. 191:1661-1674, 2000), or *P. gingivalis* LPS (Pulendran et al., J. Immunol. 167:5067-5076, 2001).

Examples of bacterial lipopeptides include bacterial cell wall lipopeptides which differ in their fatty acid chain of the N-terminal cysteines, such as diacylated and triacylated lipopeptides. For example, diacylated lipopeptides include Macrophage Activating Lipopeptide 2 kilo-Dalton from *Mycoplasma fermentans* or fragments thereof or synthetic analogues (e.g., MALP2, Pam2CSK4, Pam2CGNNDESNISFKEK, and Pam2CGNNDESNISFKEK-SK4). The triacylated lipopeptides include Pam3cys {S-[2,3-bis(palmitoyloxy)-(2-RS)-propyl]-N-palmitoyl-R-Cys-S-Ser-Lys4-OH)} (Takeuchi, et al., 2001 International Immunology 13:933-940).

In an embodiment, the agonist specifically effects TLR-2 or a receptor(s) bound by SEA (with respect to SEA, *see* MacDonald et al., J. Immunol. 167:1982-1988, 2001). Here too, the agonist can be, but is not limited to, a natural ligands, a biologically active fragment thereof, or a small or synthetic molecule. Other useful agonists may include a non-toxic form of cholera toxin (Braun et al., J. Exp. Med. 189:541-552, 1999), certain forms of *Candida albicans* (d'Ostiani et al., J. Exp. Med. 191:1661-1674, 2000), or *Porphyromonas gingivalis* LPS (Pulendran et al., J. Immunol. 167: 5067-5076, 2001). These agents fail to induce IL-12(p70) and stimulate Th2-like responses.

The agonists can be agonists of TLR-4 (which bias the immune response toward the Th response, e.g. TH1) include Taxol, fusion protein from Rous sarcomavirus, envelope proteins from MMTV, Hsp60 from *Chlamydia pneumoniae* or Hsp60 or Hsp70 from the host. Other host factors that agonize TLR-3 include the type III repeat extra domain A of fibronectin, oligosaccharides of hyaluronic acid, polysaccharide fragments of heparan sulfate, and fibrinogen. A number of synthetic compounds serve as agonists of TLR-7 (*e.g.*, imidazoquinolin (imiquimod and R-848), loxoribine, bropirimine, and others that are structurally related to nucleic acids).

Additional examples of suitable active agents include inhibitors of ERK, c-Fos, Foxp3, PI3 kinase, Akt, JNK, p38, NF-Kb, STAT 1, STAT2, IRF3, IRF7, IFN-alpha signaling or combinations thereof. Suitable active agents can further include inhibitors of SOCS 1 -7 proteins. Such inhibitors can be a small molecule, or a peptide, protein or nucleic acid (e.g., siRNA or antisense).

The agonist can be an exogenous or endogenous ligand, many of which are known in the art. The novel screening methods described below, particularly those that feature detecting TLR binding or activation, can be used to identify other ligands (whether naturally occurring molecules, fragments or derivatives thereof, antibodies, other peptides or protein-containing complexes, or synthetic ligands). For example, exogenous ligands of TLR-2 include LPS (lipopolysaccharide; a component of the outer membrane of Gram-negative bacteria), yeast-particle zymosan, bacterial peptidoglycans, lipoproteins from bacteria and mycoplasmas, and GPI anchor from *Trypanosoma cruzi;* endogenous ligands include heat shock (or "stress") proteins (*e.g.*, an Hsp60 from, for example, a bacterial or mycobacterial pathogen) and surfactant protein-A. Exogenous ligands of TLR-3 include poly(I:C) (viral dsNRA); exogenous ligands of TLR-4 include LPS, and respiratory syncytial virus (endogenous ligands include stress proteins such as an Hsp60 or Hsp70, saturated fatty acids, unsaturated fatty acids, hyaluronic acid and fragments thereof, and surfactant protein-A). Flagellin is an exogenous ligand of TLR-5. CpG (cytosine-guanine repeat) DNA and dsDNA are exogenous and endogenous ligands, respectively, of TLR-9. *See* Zuany-Amorim et al., Nature Reviews 1:797-807, 2002, and Takeda et al., Ann. Rev. Immunol. 21:355-376, 2003.

Additional examples of suitable active agents include (a) an agent that inhibits the expression or activity of an AP-1 transcription factor in a dendritic cell; (b) a dendritic cell treated in culture with an agent that inhibits the expression or activity of an AP-1 transcription factor, or (c) syngeneic T cells stimulated in culture with dendritic cells treated as described in (b). The transcription factor can include c-fos, fos-B, Foxp3, or c-jun, and the agent that inhibits expression (of the transcription factor or of any component of the pathways described herein (these components are known in the art)) can be an antisense oligonucleotide or an RNAi molecule that specifically inhibits c-fos, fos-B, Foxp3, or c-jun expression (or the expression of a kinase, phosphatase, or other component of the signaling pathways). The inhibitory active agents discussed in the context of the present biodegradable particles can also be antibodies (or variants thereof (*e.g.,* single-chain antibodies or humanized antibodies); preferably the antibodies are monoclonal antibodies).

In another embodiment, the active agent is an antagonist (e.g., inhibitor or suppressor) of an intracellular pathway that impairs TLR2 signaling or activation. The antagonists include gram negative LPS, Taxol, RSV fusion protein, MMTV envelope protein, HSP60; HSP70, Type III repeat extra domain A of fibronectin, oligosaccharides of hyaluronic acid, oligosaccharide fragments of heparan sulfate, fibrinogen and flagellin (for a review see K Takeda, et al., 2003 Annu. Rev. Immunol. 21:335-376).

In an additional embodiment, the active agent is an antagonist of an intracellular pathway that impairs SEA signaling or activation. In one other embodiment, the molecule is an antagonist of a JNK 1/2 pathway. In another embodiment, the molecule is CpG DNA which activates p38 and ERK (A-K Yi, et al., 2002 The Journal of Immunolgy 168:4711-4720).

In another embodiment, the active agent is an inhibitor of ERK ½ which can inhibit maturation of dendritic cells and thus enhancing an IL12 and Th1 response. Examples of the molecule include but are not limited to PD98059 and U0126 (A. Puig-Kroger, et al., 2001 Blood 98:2175-2182).

In another embodiment, the active agent inhibits c-fos signaling thus enhancing an IL12 and Th1 response. Such molecules include a DEF domain mutant of c-fos or any polypeptide having a DEF domain mutation (L.O. Murphy, et al., 2002 Nature Cell Biology 4:556-564 and Supplementary information pages 1-3), including: rat Fra-1, and Fra-2; mouse FosB, JunD, c-Jun, c-Myc, and Egr-1; and human JunB, N-Myc, and mPer1.

Active agents can include therapeutic genes. Examples of therapeutic genes include suicide genes. These are genes sequences, the expression of which produces a protein or agent that inhibits tumor cell growth or tumor cell death. Suicide genes include genes encoding enzymes, oncogenes, tumor suppressor genes, genes encoding toxins, genes encoding cytokines, or a gene encoding oncostatin. The purpose of the therapeutic gene is to inhibit the growth of or kill cancer cell or produce cytokines or other cytotoxic agents which directly or indirectly inhibit the growth of or kill the cancer cell.

Suitable oncogenes and tumor suppressor genes include neu, EGF, ras (including H, K, and N ras), p53, Retinoblastoma tumor suppressor gene (Rb), Wilm's Tumor Gene Product, Phosphotyrosine Phosphatase (PTPase), and nm23. Suitable toxins include Pseudomonas exotoxin A and S; diphtheria toxin (DT); E. coli LT toxins, Shiga toxin, Shiga-like toxins (SLT-1, -2), ricin, abrin, supporin, and gelonin.

Active agents can include enzymes. Suitable enzymes include thymidine kinase (TK), xanthine-guanine phosphoribosyltransferase (GPT) gene from E. coli or E. coli cytosine deaminase (CD), or hypoxanthine phosphoribosyl transferase (HPRT).

Active agents can include cytokines. Suitable cytokines include interferons, GM-CSF, interleukins, tumor necrosis factor (TNF) (Wong G, et al., Science 1985; 228:810); WO9323034 (1993); Horisberger M. A., et al., Journal of Virology, 1990 Mar, 64(3):1171-81; Li YP et al., Journal of Immunology, Feb. 1, 1992, 148(3):788-94; Pizarro T. T., et al. Transplantation, 1993 Aug., 56(2):399-404). (Breviario F., et al., Journal of Biological Chemistry, Nov. 5, 1992, 267 (31):22190-7; Espinoza-Delgado I., et al., Journal of Immunology, Nov. 1, 1992, 149(9):2961-8; Algate P. A., et al., Blood, 1994 May 1, 83(9):2459-68; Cluitmans F. H., et al., Annals of Hematology, 1994 Jun., 68(6):293-8; Martinez O. M., et al., Transplantation, 1993 May, 55(5):1159-66;

Active agents can include Growth factors. Growth factors include Transforming Growth Factor-alpha. (TGF-alpha.) and beta (TGF-beta), cytokine colony stimulating factors (Shimane M., et al., Biochemical and Biophysical Research Communications, Feb. 28, 1994, 199(1):26-32; Kay A. B., et al., Journal of Experimental Medicine, Mar. 1, 1991, 173(3):775-8; de Wit H, et al., 1994 Feb., 86(2):259-64; Sprecher E., et al., Archives of Virology, 1992, 126(1-4):253-69).

The active agents of the invention can be can be naturally occurring, synthetic, or recombinantly produced, and includes, but are not limited to, any microbial or viral component or derivative thereof, including any component that is part of the structure of, or is produced by, the microbial cell or virus including, but not limited to, a cell wall, a coat protein, an extracellular protein, an intracellular protein, any toxic or non-toxic compound, a carbohydrate, a protein-carbohydrate complex, or any other component of a microbial cell or virus. The microbial cell or virus can be pathological.

The invention provides methods for producing the particles of the invention. In one embodiment, the method comprises the steps of a) forming a hydrophobic polymer of a ketal and a diol or an unsaturated alcohol; b) forming a polymer particle of the polymer of a) in the presence of one or more active agents and thereby encapsulating the agent (s). Examples of suitable chemistries for forming the hydrophobic polymer of a ketal and a diol or an unsaturated alcohol include acetal exchange reaction using single or double emulsions and acyclic diene metathesis (Heffernan MJ and Murthy N., 2005 Bioconjug. Chem. 16(6) :1340-2; Jain RA., 2000 Biomaterials. 21(23):2475-90; Wagener K. B. and Gomez F. J., "ADMET Polymerization", in Encyclopedia of Materials: Science and Technology, E.J. Kramer and C. Hawker, Editors, Elsevier, Oxford, 5, 48 (2002)).

Suitable examples of ketals for this method include, but are not limited to, 2,2-dimethoxypropane, 2,2-dimethoxybutane, 1,1-dimethoxycyclohexane or dimethoxyacetophenole. Also in the scope of the invention are ketal polymers including aliphatic, cycloaliphatic or aromatic ketals containing one or more hetero-atom, such as nitrogen, sulfur, oxygen and halides.

In accordance with the practice of the invention, the diol can be any of alkyl, aryl and cycloalkyl diols.

Suitable examples of diols include, but are not limited to; 1,4-benzenedimethanol, 1,4-cyclohexanedimethanol, 1,5-pentane diol, 1,4-butane diol or 1,8-octave diol.

Micelles of the invention

The present invention further provides a biodegradable crosslinked micelle comprising multiple polymers. The polymers can be crosslinked by an external crosslinking agent. External cross linking agents as used herein are agents which are not introduced into the polymer chain. The advantage of using of external agents is the faster crosslinking reaction compared to a reaction wherein only crosslinkable moieties within the polymer are used. The external crosslinking agent also reduces the probability that the encapsulated protein will be destroyed.

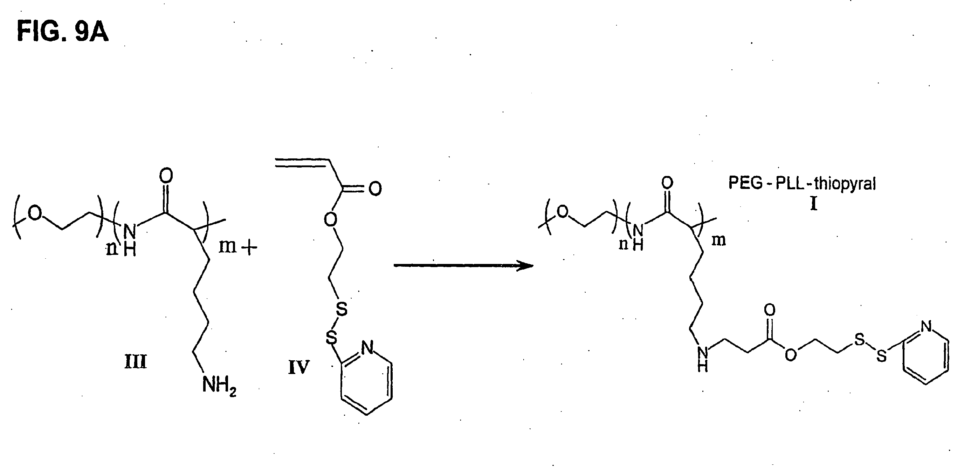

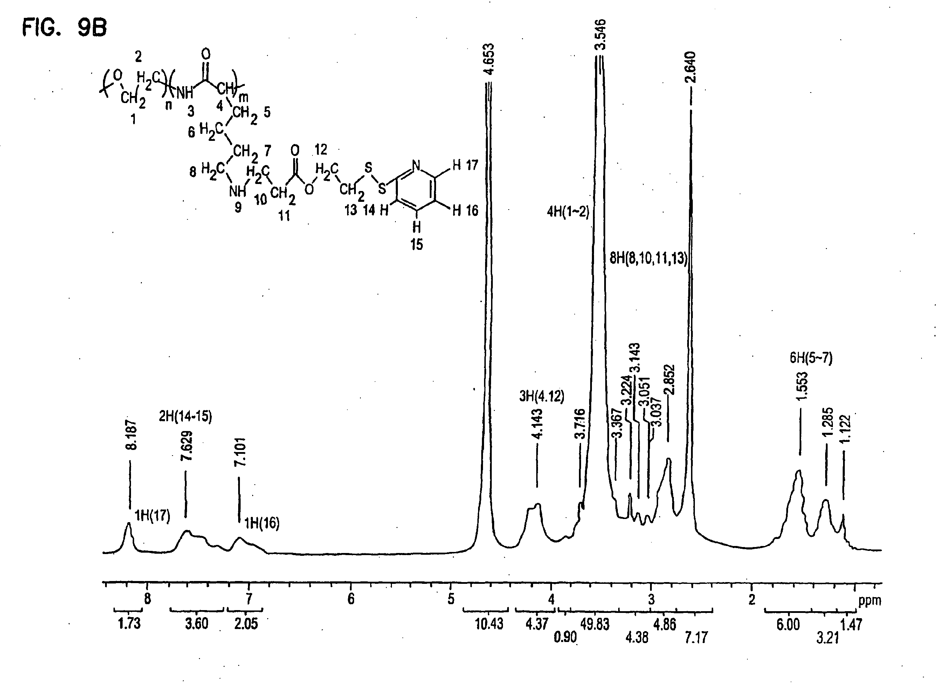

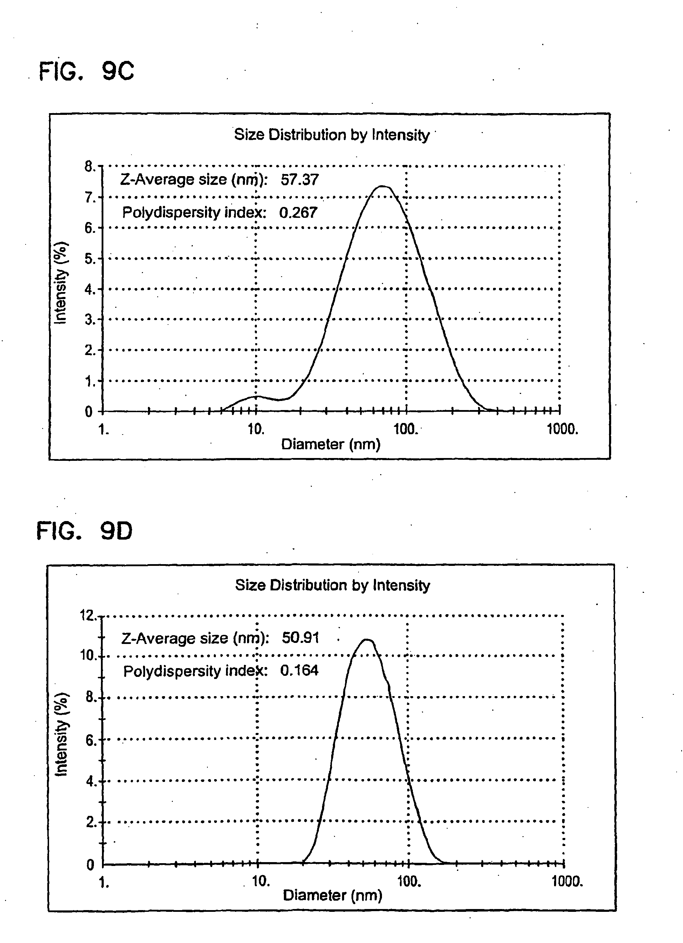

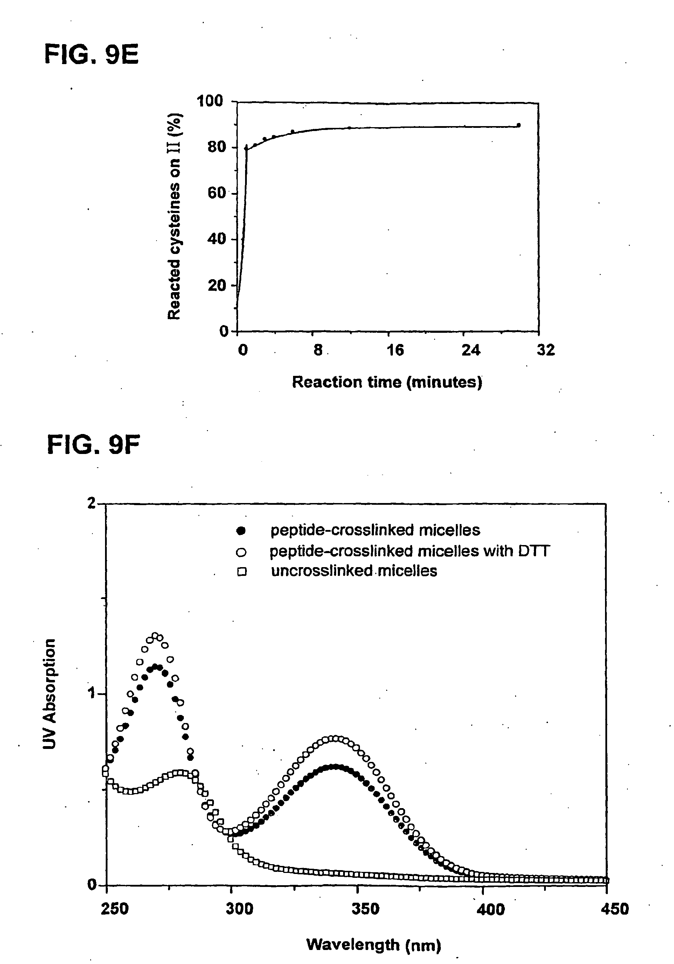

In one aspect of the present invention, suitable crosslinking agents are compounds which comprise at least two thiol groups. Examples of suitable crosslinking agents include, but are not limited to, ethylene glycol dithiol, aliphatic dithiols, dithiols which are connected by ketal linkages, and diamine containing molecules. The advantage of thiol groups is the sensitivity to reducing conditions, thus enabling an easy degradation of the micelle. Other crosslinking strategies include, but are not limited to, crosslinking by amines, esters, carbonates; thioesters, Schiff bases, vicinal diols, alkenes and alkynes, ketals, ketals orthoesters, thio-ketals, thio-orthoesters, sily-ketals, phenyl boronic acid-diol complexes, carbon-carbon bonds, sulfones, phosphate containing functional groups, azides, enzyme cleavable linkages, and urethanes, with Schiff bases, thiols and ketones being preferred in one embodiment of the present application (O'Reilly et al., 2005 Chem. Mater., 17(24):5976-5988; Hanker et al., 2005 Science 309(5738):1200-05; Le, Z. et al., 2005 Langmuir 21(25):11999-12006; Example 1, Figure 9).

In another aspect of the present invention, the external crosslinking agent comprises an antigen. Examples of suitable antigens include, but are not limited to, proteins or peptides. The antigens can be naturally occurring, chemically synthesized or recombinantly made. Specific examples of suitable protein or peptide antigens include, but are not limited to, HIV antigens such as gp120 protein (or fragment thereof), TAT protein (or fragment thereof), NEF protein (or fragment thereof), HCV protein (or fragment thereof), and env protein (or fragment thereof). A preferred antigen includes a gp120 peptide antigen chemically synthesized and modified to contain four additional cysteine residues to create therein additional disulfide bonds. Any antigen having crosslinkable groups can be used. Antigens not having or having few crosslinkable groups can be modified (e.g., chemically modified) to comprise crosslinkable thiol groups, azides, alkynes, amines, maleimides, vinyl sulfones, ketones, hydrazines and thioesters.

Polymers to be used for micelle formation can be homopolymers or copolymers, such as block copolymers or graft polymers. Examples of suitable polymers include, but are not limited to, PEG block copolymers, such as PEG-polyamino acids, for example, PEG-polylysine, PEG-polyglutamic acid, PEG-polyaspartic acid or PEG-polyarginine;

PEG polyesters, PEG-polyurethane, PEG-PPO, modified or unmodified, PEG-polyacrylate or PEG-polymethacrylate, synthesized by atom transfer polymerization, where the PEG acts as an initiator. To facilitate polymer crosslinking, the polymer can be modified to include chemical groups including, but not limited to, amines, esters, carbonates, thioesters, Schiff bases, vicinal diols, alkenes and alkynes, ketals, ketals orthoesters, thio-ketals, thio-orthoesters, sily-ketals, phenyl boronic acid-diol complexes, carbon-carbon bonds, sulfones, phosphate containing functional groups, azides, enzyme cleavable linkages, and urethanes, with Schiff bases, thiols and ketones being preferred in one embodiment of the present invention. These groups can be introduced via chemical reactions known in the art, such as, among others, Michael addition or acylation (Example 1, Figure 9). In one preferred embodiment the modified polymer is PEG-polylysine thiopyridal (Figure 9).

The polymethacrylate or polyacrylate block can contain modifications to allow for assembly with vaccine components and crosslinking. For example a polyacrylate block can be a block copolymer consisting of polydimethylamino-acrylate-poly-glycidyl acrylate. Homopolymer of random copolymers composed of various acrylate or methacrylate monomers capable of forming micelles with vaccine components are also in the scope of the present invention.

In another aspect of the present invention the micelle can further comprise one or more active agents. Examples of suitable active agents are found herein, underline_supra.

In accordance with the practice of the invention, the interaction between the micelle and the active agent can be electrostatic or hydrophobic or can occur due to hydrogen bond formation or molecular recognition depending on the type of polymer and agent. The surface of this micelle can be modified to contain targeting groups such as, for example, antibodies against dendritic cells, or proteins which stimulate subsets of dendritic cells, or macrophages such as CD40L, DEC-205, CD11c, langerin, MARCO, 33D1 etc.

The micelles can be produced in a two step process. First, the polymers of interest can be contacted with a liquid. (polar or nonpolar liquid depending on the polymer to be used) under appropriate conditions so as to form a micelle. After micelle formation, the micelle can be crosslinked with an external crosslinking agent to produce the biodegradable micelle of the invention.

In one embodiment, the micelle is designed to deliver peptide antigens and immunomodulatory molecules to antigen-presenting cells (APCs). In this embodiment, the micelle comprises immunomodulatory molecules, peptide antigens and a copolymer. The peptide antigen acts as a crosslinker which allows the peptide antigen to be efficiently encapsulated into the peptide crosslinked micelles (PCMs) and also stabilizes them against degradation by serum components

In another embodiment, the micelle can be used to encapsulate peptide or protein antigens together with immunomodulatory agents including multiple TLR ligands, or molecules such as synthetic compounds or siRNA that modulate signaling networks within cells (e.g., dendritic cells or other antigen presenting cells). The micelle targets dendritic cells and macrophages through their nanometer dimensions, as such cells robustly internalize nanometer sized materials, through phagocytosis. The micelles are crosslinked by crosslinking agents comprising disulfide linkages, which should stabilize them against decomposition induced by serum proteins. In the further embodiment of the invention, the micelle has a size of 5 to 50 microns.

After phagocytosis, the biodegradable particles and micelles of the invention will breaks down, and the encapsulated material such as peptide or antigens, and immune stimulatory agents [e.g: ISS DNA, TLR 7/8, TLR 3 ligands such as ss RNA, TLR 2 ligands, and inhibitors of regulatory pathways such as the ERK, c-Fos or Foxp3 pathway], will be released into the dendritic cell, or macrophage, and the immunomodulatory agent will induce the antigen-presenting cells, to secrete a variety of cytokines. This combination of signals will result in the optimal activation of T cells, and inhibition of regulatory T cells and dendritic cells.

The micelles of the invention can include active agents such as vaccines composed of antigens from the relevant pathogen, together with immune modulatory agents [e.g: ISS DNA, TLR 7/8 ligands such as ss RNA, TLR 2 ligands, and inhibitors of regulatory pathways such as inhibitors of ERK, c-Fos or Foxp3, PI3 kinase, Akt, SOCS 1-7 proteins, or siRNA molecules or antisense molecules that inhibit such regulatory pathways]

The invention also provides methods for reversibly modifying proteins so that they have the appropriate charge to be encapsulated in the micelles. This strategy is based on reacting the amine groups of said protein with a compound, generating additional negative charges for every amine group, and rendering the protein negative. The modified protein will then be encapsulated in the micelle, crosslinked and then the pH will be reduced in order to remove the inserted compound form the protein. In one embodiment of the invention, the compound is amine groups is cis-aconityl. This group adds to negative charges to each amine group and can be released at ph 4.0.

Examples for targeting strategies include synthesizing a heterobifunctional PEG that has a DNA binding domain at one end and another end that can attached to a protein. This PEG chain is then attached to a protein and then assembled into a preformed micelles that contains immunostimulatory DNA. Examples of DNA binding domains include acridine or polyacridines. Examples of targeting ligands include galactose, mannose phosphate, mannose, peptides, and antibodies.

Further modifications of the biodegradable particles and micelles of the invention

The biodegradable particle or micelles can be further modified to incorporate antibodies or other molecules which target (1) specific receptors on particular subsets of DCs or macrophages or monocytes, including Langerhans cells, dermal DCs, myeloid DCs, plasmacytoid DCs or (2) specific receptors on antigen-presenting cells, such as DEC205, Langerin, DC-SIGN, dectin-1, 33D1, MARCO.

## METHODS FOR USING THE COMPOSITIONS OF THE INVENTION

The invention further provides methods for delivering the active agents of the invention, via the particles of the invention or the micelles of the invention, to a subject in order to, for example, deliver active agents so as to treat the subject suffering from a disease (e.g. alleviate symptoms associated with the disease). The disease can be any of HIV, malaria, . TB, SARS, anthrax, Ebola, influenza, avian influenza and HCV. Further disease or disorder can be any of an infectious disease, autoimmune disease, allergic disease, disorder or complications associated with transplantation, diabetes and cancer. Examples of autoimmune disease include lupus, rheumatoid arthritis, psoriasis, asthma and COPD.

The active agents are delivered through the particles of the invention by various administration means including, but not limited to, intravenous, subcutaneous, intramuscular, oral and inhalation means. The most effective mode of administration and dosage regimen for the compositions of the present invention depends upon the exact location of the disease or disorder being treated, the severity and course of the disease or disorder, the subject's health and response to treatment and the judgment of the treating physician. Accordingly, the dosages of the molecules should be titrated to the individual subject.

The interrelationship of dosages for animals of various sizes and species and humans based on $mg/m^2$ of surface area is described by Freireich, E. J:, et al. Cancer Chemother., Rep. 50 (4):219-244 (1966). Adjustments in the dosage regimen maybe made to optimize the tumor cell growth inhibiting and killing response, e.g., doses may be divided and administered on a daily basis or the dose reduced proportionally depending upon the situation (e.g., several divided dose may be administered daily or proportionally reduced depending on the specific therapeutic situation). It would be clear that the dose of the compositions of the invention required to achieve treatment may be further reduced with schedule optimization.

As used herein, the term "subject" may include a human, any animal such as equine, porcine, bovine, murine, canine, feline, and avian subject, a cell or a cell tissue. In accordance with the practice of the invention the active agents can be delivered or administered to the subject (using the compositions of the invention) before, after, or during the onset of the disease or disorder.

The invention provides methods for regulating an immune response by administering active agents via the particles or micelles of the invention. For example, in the methods of the invention, an immune response can be biased towards a Th immune response in a TLR-dependent manner by delivering or administering to a subject a particle or micelle of the invention containing a desired active agent. In one embodiment, a TLR-expressing cell is contacted with an agent (delivered by the particle or micelle of the invention) that effects a bias towards a Th immune response (e.g., a Th0 Th2 or T regulatory cell immune response). For example, the agent (*e.g.*, a natural ligand, a biologically active fragment thereof, or a small or synthetic molecule) that activatesTLR-2, ERK ½, or c-fos.

As noted above, the immune response can be regulated or modulated (e.g., increase biasing or decrease biasing toward a Th immune response) at a point in the signaling pathway downstream from receptor activation (*e.g.*, downstream from TLR binding or downstream from TLR activation or recognition). Thus, the patient can also be treated with an active agent or combination of active agents (delivered using the compositions of the invention) that bias the immune response by acting intracellularly on the elements of the downstream signaling pathway.

The present invention provides methods for biasing towards a Th2 immune response by inducing cell signaling (e.g., activation) of any of the MAP kinase pathways, including an ERK ½ pathway using a desired active agent delivered using the compositions of the invention. An induced MAP kinase pathway can be characterized by an increase in the amount and/or duration of phosphorylated components of the MAP kinase pathways, including ERK ½.

In another embodiment of the methods of the invention, an active agent can be delivered, via the compositions of the invention, which modulates an ERK ½ MAP kinase, pathway so as to regulate a TH2 immune response. In this embodiment, as an example, an agonist of a TLR (e.g., TLR-2) induces phosphorylation of ERK ½ so as to enhance a TH2 immune response. In yet another embodiment of the methods of the invention, an active agent can be delivered via the compositions of the invention so as to modulate a c-FOS pathway in the cell thereby regulating a TH2 immune response. In this embodiment, as an example, an agonist of a c-fos pathway induces expression of c-fos and/or phosphorylation of c-fos so as to enhance a TH2 immune response. Additionally, in yet a further embodiment of the methods of the invention, an active agent can be delivered via the compositions of the invention, so as to modulate a Th2 immune response by affecting TLR2 or its downstream signaling pathway elements such as ERK ½ may kinase pathway and a c-FOS pathway. For example, the active agent, delivered via the compositions of the invention, can be used to modulate production or activity of IL-10 (for example increase production or upregulate of IL-10).

In one embodiment, the methods for biasing towards a Th2 immune response includes decreasing or inhibiting signaling of p38 and/or JNK pathway(s) which mediate (e.g., inhibit) IL12 production and thus biasing against a Th1 response by administering an active agent delivered via the compositions of the invention. In another embodiment, the methods for biasing towards a Th2 immune response includes decreasing or inhibiting the amount of phosphorylated p38 and/or JNK, or decreasing or inhibiting the duration of phosphorylation of p38 and/or JNK which mediate (e.g., inhibit) IL12 production and thus biasing against a Th1 response by administering micelles or particles of the invention containing a desired active agent (or combination thereof).

The present invention also provides methods for biasing towards a Th1 immune response by inducing cell signaling (e.g., activation) of any of the MAP kinase pathways, including a p38 and/or JNK pathway by administering an active agent delivered via the compositions of the invention. An induced p38 and/or JNK pathway can be characterized by an increase in the amount and/or duration of phosphorylated components of the MAP kinase pathways, including p38, and/or JNK.

In one embodiment, the methods for biasing towards a Th1 immune response includes decreasing or inhibiting signaling of ERK ½ and/or c-fos pathway(s) by administering an active agent delivered via the compositions of the invention. In another embodiment, the methods for biasing towards a Th1 immune response includes decreasing or inhibiting the amount of phosphorylated ERK ½ and/or c-fos, or decreasing or inhibiting the duration of phosphorylation of ERK ½ and/or c-fos by administering an active agent delivered via the compositions of the invention.

Additionally, the invention provides methods for regulating a TH2 immune response which comprises contacting a T cell (e.g., a naïve T cell) with a TLR-positive cell (such as a DC) treated in culture with a TLR agonist (e.g., TLR-2 agonist), delivered via the compositions of the invention, which activates an ERK ½ pathway and/or which activates c-fos or c-fos pathway.

Additionally, the invention provides methods for regulating a TH1 immune response which comprises contacting a T cell (e.g., a naive T cell) with a TLR-positive cell treated in culture with a TLR agonist (e.g., TLR-4 agonist), delivered via the compositions of the invention, which activates a p38 pathway and/or a JNK pathway.

The present invention provides methods for treating a subject having an immune-related condition or disease (e.g., allergies, autoimmune disease; and other immune-related conditions including cancer), comprising administering to the subject any of the agents of the invention, delivered via the compositions of the invention, in an amount effective to bias towards or against a Th1, T2 or Th0 immune response. The subject can be bovine, porcine, murine, equine, canine, feline, simian, human, ovine, piscine or avian.

In one embodiment, a subject having a condition or disease associated with an exhuberant Th2 response is treated with an agent of the invention, delivered via the compositions of the invention, that activates cell signaling in the subject so as to bias towards a Th1 immune response. Disease characterized by exhuberant Th2 response include, but are not limited to allergy, asthma, and chronic obstructive pulmonary disease (COPD (*e.g.*, emphysema or chronic bronchitis).

In another embodiment, a subject having a condition or disease associated with an exhuberant Th2 response is treated with a molecule that inhibits biasing towards a Th2 immune response, delivered via the compositions of the invention.

In one embodiment, a subject having a condition or disease associated with an exuberant Th1 response is treated with a agents of the invention, delivered via the compositions of the invention, that activates cell signaling in the subject so as to bias towards a Th2 immune response. Disease characterized by exhuberant Th1 response include, but are not limited to diabetes, rheumatoid arthritis, multiple sclerosis, psoriasis, and systemic lupus erythematosis.

In another embodiment, a subject having a condition or disease associated with an exuberant Th1 response is treated with an active agent, delivered via the compositions of the invention, that inhibits biasing towards a Th1 immune response.

The following examples are presented to illustrate the present invention and to assist one of ordinary skill in making and using the same. The examples are not intended in any way to otherwise limit the scope of the invention.

**EXAMPLES**

EXAMPLE 1

*Synthesis of antigen containing crosslinked micelles*

Crosslinked micelles that contain the protein antigen Ovalbumin and immunostimulatory DNA were synthesized in a two step process. First, micelles were formed between the cationic block copolymer, PEG-polylysine-thiopyridal and negatively charged FITC-Ovalbumin (FITC-OVA) and immunostimulatory DNA (ISS-DNA). These micelles contained a 10mg/ml concentration of PEG-polylysine-thiopyridal, a 0.5 mg/ml concentration of FITC-OVA and a 0.5 mg/ml concentration of ISS-DNA. The micelles were allowed to form for one hour and were then crosslinked with 0.4mg/ml of dithio-ethylene glycol. The crosslinking reaction was monitored by U.V. activity (342nm), and indicated that the thiopyridal groups had been quantitatively reacted after 1 hour at room temperature. The encapsulation efficiency of FITC-OVA in the micelles was determined by centrifuging the micelles through a 100kD spin-filter (centricon) and analyzing the recovered solution for FITC fluorescence (excitation 494nm, emission 510nm). This indicated that over 95% of the FITC-OVA was encapsulated in the micelles.

EXAMPLE 2

*Synthesis of polyketal particles (single emulsion method for delivery of hydrophobic drugs)*

Particles were synthesized with, poly(1,4-phenylene-acetone dimethylene ketal) using an oil-in-water emulsion method. Briefly, 10 mg of 2, 1mg of the ERK inhibitor UO126 and 0.1 g of chloro-methyl fluorescein diacetate (CMFDA), were dissolved in 0.5 mL of $CHCl_3$ (with 0.1% triethylamine). This solution was then added to 5 mL of pH 9 buffer (10 mM $NaHCO_3$) containing 2mg/ml polyvinyl alcohol (PVA, 31-50 kDa, Aldrich). The oil-water mixture was shaken briefly and then sonicated for 2 to 3 min at 40 watts (Branson Sonifier 250) to form a fine oil/water emulsion. The emulsion was stirred under $N_2$ flow for at least 3 h to evaporate the solvent and produce a particle suspension. Particle sizes were analyzed by dynamic light scattering (DLS) and indicated that the average diameter was 282 nm.

EXAMPLE 3

*Synthesis of polyketal particles (double emulsion method for synthesis of hydrophilic drugs)*

Polyketal particles (PKNs) containing FITC-ovalbumin were fabricated using a double emulsion method. First, 20 mg of poly(1,4-phenylene acetone dimethylene ketal) (PPADK) dissolved in 500 $\mu$L of chloroform was added to 100 $\mu$L of FITC-Ova solution (~0.7 mg). This mixture was sonicated at 40 watts for minute to form the primary emulsion. Next, 5 mL of 0.2% w/v polyvinyl alcohol (PVA, Aldrich) in 10 mM pH 9 sodium phosphate buffer was added, and this mixture was sonicated at 40 watts for at least 1 minute to form the secondary emulsion. The emulsion was mixed under nitrogen ventilation for 4 hours, after which the volume was made up to 5 mL with buffer. Two batches of PKNs containing FITC-Ova were prepared in this manner, as well as two batches of plain PKNs (without FITC-Ova). The PKN suspensions were stored at 4°C. Particle sizing was determined by dynamic light scattering (DLS). The two batches of FITC-Ovalbumin-loaded PKNs had effective diameters of 426 nm and 462 nm, and the empty PKN batches were 321 nm and 347 nm.

*Labeling of FITC-Ova*

Chicken egg albumin (ovalbumin) was labeled with fluorescein as follows. Ovalbumin was dissolved at 10 mg/mL in 200 mM pH 9 $NaHCO_3$ buffer. Fluorescein isothiocyanate (FITC) was dissolved at 10 mg/mL in DMSO. Next, 2.5 mL of ovalbumin solution (25 mg, 0.56 mmol) was mixed with 50 $\mu$L of FITC/DMSO (0.5 mg, 1.28 mmol) for at least 1 hour at 30°C. The product was filtered in a Sephadex PD-10 column to remove the free dye; the column was loaded with 2.5 mL of product and was eluted with 2.5 mL water. The resulting ovalbumin concentration was approximately 7 mg/mL. The degree of labeling was calculated to be 1.09 by measuring the absorbance of FITC at 497 nm and using the estimated concentration of ovalbumin.

*Determination of FITC-Ova encapsulation in PKNs*

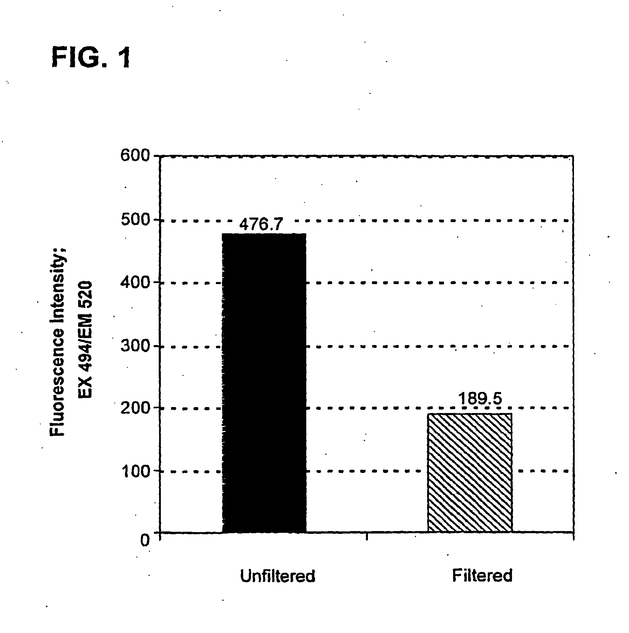

The two FTTC-Ova PKN batches were diluted by 5-fold in water, and a portion of each diluted sample was filtered through a 0.1 $\mu$m Supor syringe filter (Pall Acrodisc). The filtered and unfiltered samples were further diluted by 10-fold into pH 9 sodium phosphate buffer, and the fluorescence was measured with 494 nm excitation wavelength and 520 nm emission wavelength. (Figure 1)

The encapsulation efficiency was calculated using the fluorescence intensity of the filtered and unfiltered samples, as follows:

$$EncapsulationEfficiency = \left(1 - \frac{Filtered}{Unfiltered}\right) \times 100\% = \left(1 - \frac{189.5}{476.7}\right) \times 100\% = 60\%$$

EXAMPLE 4

*Synthesis and degradation of ketal-backbone polymer (polyketal)*

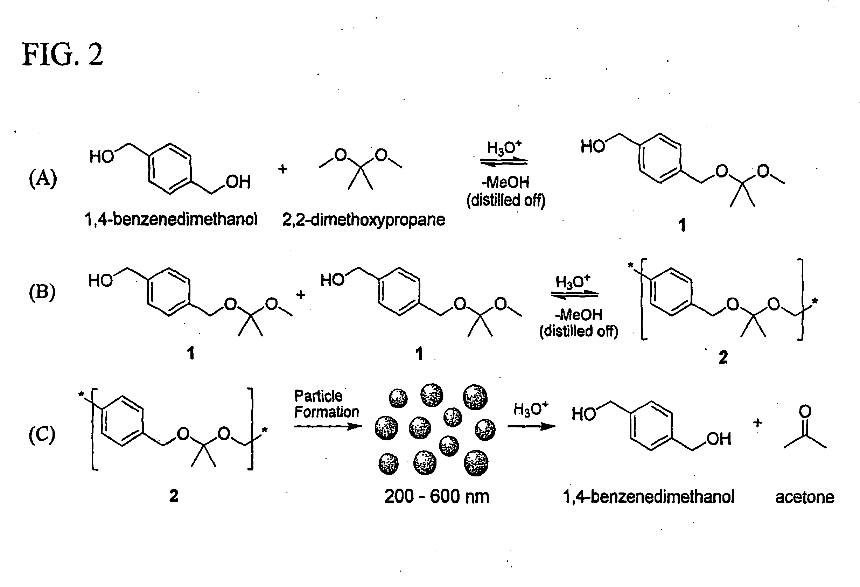

Figure 2 shows: (A) Ketal exchange reaction between 1,4-benzenedimethanol and 2,2-dimethoxypropane to produce the ketal intermediate 1. (B) Stepwise polymerization of 1 to produce polyketal 2. Reaction steps A and B are driven forward by distilling off the methanol byproduct. (C) Formation of drug-loaded particles by the solvent evaporation method. Particles exhibit pH-sensitive degradation into low molecular weight excretable compounds.

*Synthesis*

The Polyketals are synthesized via a new polymerization strategy based on the ketal exchange reaction (14). This reaction is generally used to introduce protecting groups onto low molecular weight alcohols and has not been used previously to synthesize polymers. However, we demonstrate here that the ketal exchange reaction can be used to synthesize an acid sensitive polymer by simply reacting 2,2-dimethoxypropane (DMP) with a diol. We propose that the polymerization occurs through the reaction mechanism in Figure 2. The ketal exchange reaction is an equilibrium reaction involving protonation of DMP followed by nucleophilic attack by the alcohol. This equilibrium is shifted toward formation of the ketal intermediate 1 by distilling off the methanol byproduct. As the reaction proceeds, molecules of 1 combine in a stepwise manner to form 2.

A representative polymerization of DMP and 1,4-benzenedimethanol (BDM) gave the polyketal 2 with a 48% yield. The polymerization was carried out in a 25 mL two-necked flask connected to a short-path distilling head. BDM (1.0 g, 7.3 mmol, Aldrich) dissolved in 10 mL warm ethyl acetate was added to 10 mL distilled benzene kept at 100°C. Re-crystallized *p*-toluene sulfonic acid (5.5 mg, 0.029 mmol, Aldrich) dissolved in 550 μL ethyl acetate was then added. After allowing the ethyl acetate to distill off, distilled DMP (900 μL, 7.4 mmol, Aldrich) was added to initiate the reaction. Additional doses of DMP were added via a metering funnel, with each dose consisting of 2 mL benzene plus 300 to 500 μL DMP. Each dose was added over a 30 to 40 min period with a 30 min interval in between. The total duration of the reaction was 7 h. The reaction was stopped with the addition of 100 μL triethylamine and was precipitated in cold hexanes. The crude product was vacuum filtered, rinsed with ether and hexanes, and vacuum dried to yield 600 mg of white solid product (48% yield). The recovered polymer was analyzed by GPC and $^1$H NMR.

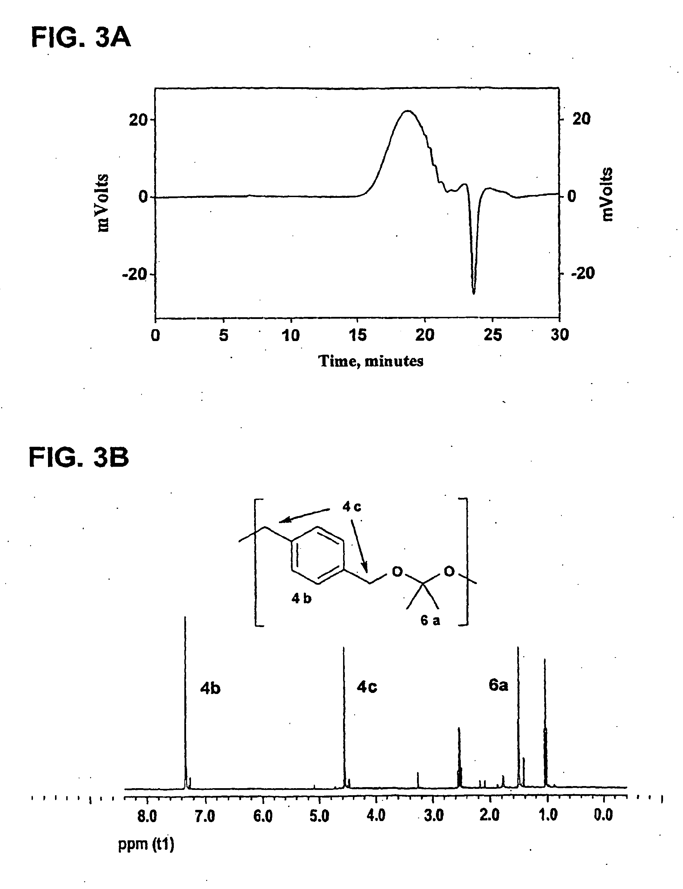

Figure 3A shows the GPC trace from one batch in which $M_w$ = 4000 was obtained, corresponding to a degree of polymerization of 22.5 repeating units, with a polydispersity index of 1.54. The $^1$H NMR spectrum (Figure 3B) confirms that the repeating unit of 2 contains a dimethyl ketal group ('6a'). Together, the GPC and $^1$H NMR data provide evidence for the successful synthesis of polyketal 2.

*Hydrolysis*

The hydrolysis kinetics of 2 were measured at pH values corresponding to lysosomes (pH 5.0) and the bloodstream (pH 7.4). The hydrolysis rates were measured by grinding polyketal 2 into a fine powder and adding it to deuterated solutions at pH 7.4 (phosphate buffer), pH 5.0 (acetate buffer), and pH 1.0 (DCl). The suspensions were stirred at 37°C and data points were taken at 3 h, 24 h, 48 h, and 72 h. Each suspension was centrifuged for 4 min at 1800 g, and the supernatant was analyzed by $^1$H NMR. The spectra contained peaks for BDM (7.24 and 4.47 ppm) and acetone (2.05 ppm). The average of the two BDM peak integrals was used to determine the relative degree of hydrolysis. The percent hydrolysis was calculated as the BDM peak average of the pH 7.4 or 5.0 sample divided by the BDM peak average of the pH 1.0 control batch.

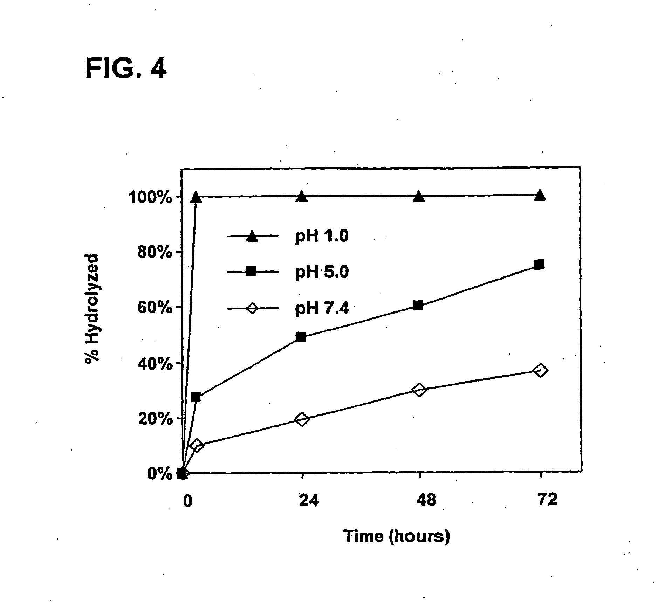

Exponential decay half-lives were calculated to be 102 h at pH 7.4 and 35 h at pH 5.0, representing a 3-fold rate increase from pH 7.4 to 5.0 (Figure 4). The pH sensitivity of 2 is significantly less than that reported by Kwon, et al. (9) for a water-soluble ketal. We hypothesize that the lower pH sensitivity of 2 is due to its water insolubility, which limits the diffusion of water and creates another rate limiting step that is insensitive to pH. The diffusion kinetics of water into materials made of 2 will be dependent on the size of the particles and we would expect smaller particles to have greater pH sensitivity than ground particles.

EXAMPLE 5

*Synthesis of micron sized particles*

Compound 2 was also used to synthesize micron sized particles. An oil-in-water emulsion method (15) was used to form the particles. Briefly, 50 mg of 2 dissolved in 1 mL $CHCl_3$ (with 0.1 % triethylamine) was added to 5 mL of 10 mM $NaHCO_3$ pH 9 buffer containing various amounts of polyvinyl alcohol (PVA, 31-50 kDa, Aldrich) as the emulsifier.

The oil-water mixture was shaken briefly and then sonicated for 2 to 3 min at 40 watts (Branson Sonifier 250) to form a fine oil/water emulsion. The emulsion was stirred under $N_2$ flow for at least 3 h to evaporate the solvent and produce a particle suspension.

Particle sizes were analyzed by dynamic light scattering (DLS) and SEM. DLS samples were prepared by diluting the particle suspension in 10 mL pH 9 buffer and allowing the larger particles to settle out. An aliquot from the liquid portion of each vial was then diluted for DLS particle sizing (Brookhaven 90Plus particle sizer). An SEM sample was made with the 0.2:1 ratio of PVA:polyketal by centrifuging the particle suspension for 10 min (5000 g, 4°C), washing with distilled water, and lyophilizing the recovered pellet.

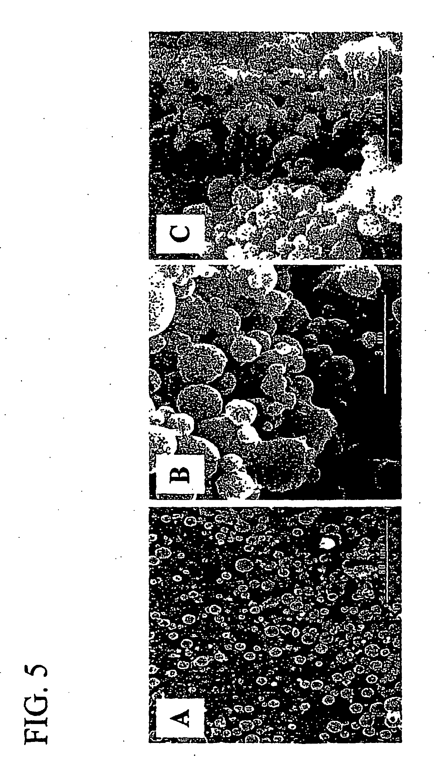

As expected, the particle size was sensitive to the ratio of PVA to polyketal. The DLS particle diameters were 520 nm, 290 nm, and 280 nm for samples containing 0.2:1, 0.8:1, and 2:1 ratios of PVA:polyketal, respectively. The SEM images of the 0.2:1 batch (Figures 5A and 5B) confirm that the polyketal does form micron sized particles, with particle size distribution ranging from 0.5 to 30 $\mu$m in diameter.

EXAMPLE 6

_Encapsulation of dexamethasone in polyketal particle_

The anti-inflammatory drug dexamethasone (Dex, Sigma) was encapsulated into particles made with 2. Dex-loaded particles were formulated using the same procedure as that described above, except that the oil phase contained a 5 mg/ml concentration of Dex and a 1:1 ratio of PVA:polyketal was used. SEM images of these particles demonstrate that they are 200-600 nm in diameter (Figure 5C). Particle sizing by DLS indicated an effective diameter of 250 nm for the Dex-loaded particle batches. The Dex encapsulation efficiency ranged between 43-53%. Control batches were prepared with polyketal/PVA only and Dex only. To measure Dex encapsulation, each particle batch was re-suspended in pH 9 buffer, and an aliquot was then further diluted. A portion was filtered through a 0.1 $\mu$m Supor membrane Acrodisc syringe filter (Pall Corp.), and the 242 nm absorbance of the filtrate was recorded with a Shimadzu UV-1700 spectrophotometer. The encapsulation efficiency was calculated as $(A_{Dex} - A_{Dexpoly})/(A_{Dex} - Ap_{oly})$, where A is the absorbance at 242 nm and the subscripts 'Poly', 'Dex', and 'DexPoly' refer to the 'Polyketal only', 'Dex only', and 'Dex + Polyketal' samples, respectively. These calculations resulted in a Dex encapsulation efficiency of 43% to 53% for various samples.

References for Examples 4-6:

(1) Ahsan, F.; Rivas, 1. P.; Khan, M. A.; Torres-Suarez, A. I. J. Controlled Release 2002, 79, 29-40.

(2) Prior, S.; Gander, B.; Blarer, N.; Merkle, H. P.; Subirá, M. L.; Irache, J. M.; Gamazo, C. Eur. J. Pharm. Sci. 2002,15,197-207.

(3) Hahn, S. K.; Jelacic, S.; Maier, R. V.; Stayton, P. S.; Hoffman, A. S. J. Biomater. Sci. Polymer Ed". 2004, 15, 1111-1119..

(4) Walter, E.; Dreher, D.; Kok, M.; Thiele, L.; Kiama, S. G.; Gehr, P.; Merkle, H. P. J. Controlled Release 2001, 76,149-168.

(5) van Apeldoorn, A. A.; van Manen, H.-J.; Bezemer, J. M.; de Bruijn, J. D.; van Blitterswijk, C. A.; Otto, C. J. Am. Chem. Soc. 2004,126, 13226-13227.

(6) Fu, K.; Pack, D. W.; Klibanov, A. M.; Langer, R. Pharm. Res. 2000,17, 100-106.

(7) Shenderova, A.; Burke, T. G.; Schwendeman, S. P. Pharm. Res. 1999,16, 241-248.

(8) Fife, T. H.; Jao, L. K. J. Org. Chem. 1965,30,1492-1495.

(9) Kwon, Y. J.; Standley, S. M.; Goodwin, A. P.; Gillies, E. R.; Fréchet, J. M. J. Mol. Pharm. 2005, 2, 83-91.

(10) Murthy, N.; Campbell, J.; Fausto, N.; Hoffman, A. S.; Stayton, P. S. Bioconjugate Chem. 2003,14, 412-419..

(11) Murthy, N.; Xu, M.; Schuck, S.; Kunisawa, J.; Shastri, N.; Fréchet, J. M. J. Proc. Natl. Acad Sci. U.S.A. 2003, 100, 4995-5000.

(12) Standley, S. M.; Kwon, Y. J.; Murthy, N.; Kunisawa, J.; Shastri, N.; Guillaudeu, S. J.; Lau, L.; Fréchet, J. M. J. Bioconjugate Chem. 2004,15,1281-1288.

(13) Gillies, E. R.; Goodwin, A. P.; Fréchet, J. M. J. Bioconjugate Chem. 2004,15, 1254-1263.

(14) Lorette, N. B.; Howard, W. L. J. Org. Chem. 1960, 25, 521-525.

(15) Panyam, J.; Williams, D.; Dash, A.; Leslie-Pelecky, D.; Labhasetwar, V. J. Pharm. Sci. 2004, 93, 1804-1814.

**Experimental details of figures 1-15, 17-19 and 22-24**

Figure 1 is a bar graph showing the fluorescence intensity of filtered and unfiltered PKNs with FITC-Ova (excitation 494 nm, emission 520 nm). The data shown is the average of two samples. The FITC-Ova encapsulation efficiency is 60%.

Figure 2 diagrammatic representation showing the synthesis and degradation of ketal-backbone polymer (polyketal). (A) Ketal exchange reaction between 1,4-benzenedimethanol and 2,2-dimethoxypropane to produce the ketal intermediate 1. (B) Stepwise polymerization of 1 to produce polyketal 2. Reaction steps A and B are driven forward by distilling off the methanol byproduct. (C) Formation of drug-loaded particles by the solvent, evaporation method. Particles exhibit pH-sensitive degradation into low molecular weight excretable compounds.

Figure 3 (A) shows a GPC trace of polyketal 2 in THF (Shimadzu SCL-10A). $M_w$ = 4000, $M_w/M_n$ = 1.54 based on a polystyrene standard (Polymer Laboratories, Inc.). Y-axis indicates relative absorbance at 262 nm. (B) shows $^1$H NMR spectrum of polyketal 2 in $CDCl_3$ (Varian Mercury Vx 400); repeating unit peaks at 7.3 ppm (4b), 4.5 ppm (4c), and 1.5 ppm (6a). Peaks at 2.5 and 1.0 are due to triethylamine added to prevent ketal hydrolysis.

Figure 4 is a line graph showing the hydrolysis kinetics of polyketal 2 (finely ground powder) at pH 1.0, 5.0, and 7.4. Exponential decay half-lives are 102 h (pH 7.4) and 35 h (pH 5.0). The pH 1.0 control batch was completely hydrolyzed before the first time point.

Figure 5 shows SEM images of particles made with polyketal 2. (A,B) Particles using 0.2:1 ratio of PVA to polyketal 2 (particle size: 0.5 - 30 $\mu$m). (C) Dexamethasone-loaded particles made using 1:1 PVA:polyketal 2 (particle size: 200 - 500 nm). Scale bars are (A) 80 $\mu$m, (B) 3 $\mu$m, and (C) 4 $\mu$m.

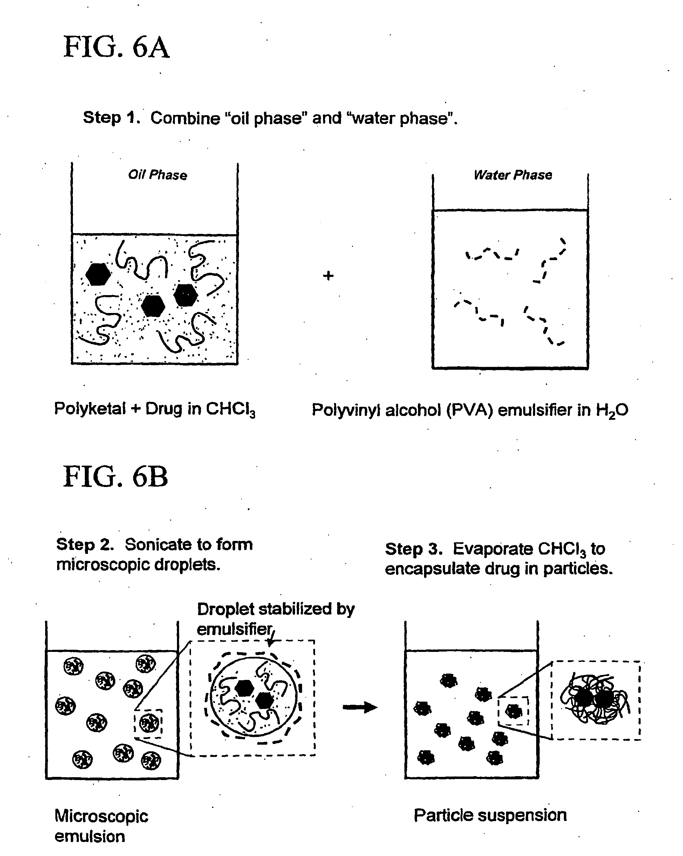

Figure 6 is a schematic representation showing particle formation. A. Step 1: Dissolve polyketal and drug into chloroform; dissolve polyvinyl alcohol in water. B. Step 2: Add chloroform solution to water and sonicate, generate micron sized droplets. Step 3: Let chloroform evaoporate, generates particles.

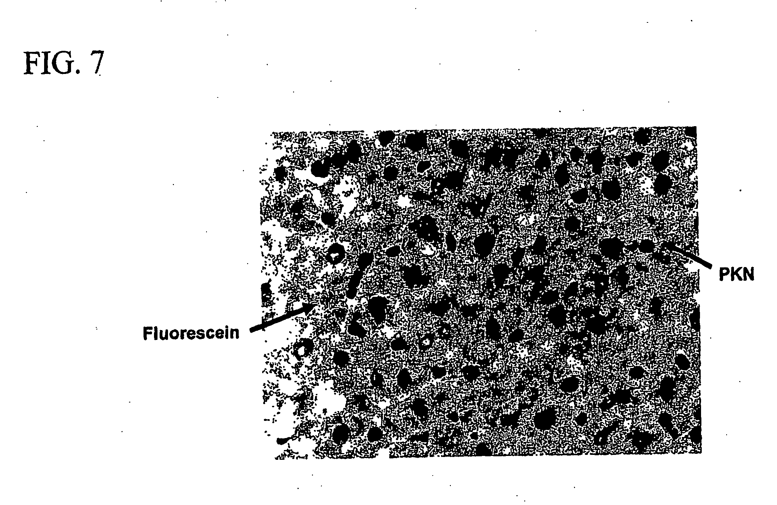

Figure 7 shows polyketal particles loaded with Fluorescein are taken up in the liver. Murine liver tissue slice showing release of fluorescein from PKNs following intravenous injection.

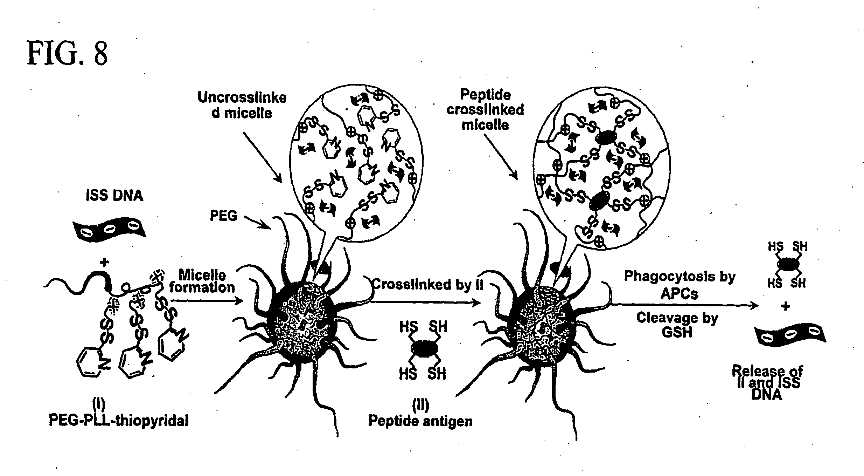

Figure 8 is a schematic representation showing peptide crosslinked micelle design and synthesis. Step 1: ISS DNA and I are mixed to form micelles (uncrosslinked micelle). Step 2: These micelles are then crosslinked with the antigenic peptide (II) to generate a delivery system that can encapsulate both immunostimulatory molecules and peptide antigens. After phagocytosis by APCs, the peptide-crosslinked micelles release their components.
An HIV peptide vaccine was synthesized using the PCM strategy with the peptide CGCRIQRGPGRAFVTIGKCGCG (II). The peptide II comes from the GP-120 protein and contains the sequence RIQRGPGRAFVTIGK, which is both a class I and II antigen. First, micelles were formed between I and ISS-DNA by mixing 0.5 mg of I with 0.1 mg of ISS DNA (5-TCCATGACGTTCCTGACGTT-3) (charge ratio was 1 to 15 (-/+)) in 0.5ml of 50mM PBS. Dynamic light scattering of these micelles using the Cumulants method indicated that they had an average diameter of 57.0 nm. These micelles were then crosslinked by adding 0.1 mg of II to the micelles (equal molar ratio of cysteines on II to thiopyridal groups on I). The peptide II was incorporated into the micelles through a disulfide exchange reaction.

Figure 9 shows synthesis and characterization of PEG-polylysine thiopyridal. A. Synthesis of PEG-polylysine thiopyridal (I). 44 $\mu$mole of PEG-Poly-$l$-lysine (with PEG = 5kd and Poly-$l$-lysine = 5,000) was dissolved in 1ml of DMF, in a 5ml round bottom flask, fitted with a stir bar (overnight stirring at room temperature was required to completely dissolve the polymer). 415 $\mu$mole of hydroxyl-ethyl thiopyridal acrylate and 58$\mu$l of triethylamine were then added to the PEG-poly-$l$-lysine solution and the reaction was allowed to run for 24 hours at room temperature. The product was isolated by precipitating the reaction solution into 15 ml of ice cold diethyl ether. The yield was 88.2%. B. 1H-NMR spectrum of PEG-PLL-thiopyridal in $D_2O$. The product of A) was analyzed by $^1$H NMR in $D_2O$. The percentage of amines alkylated was determined by comparing the peak intensity ratio of pyridine protons (-$NC_5H_4$: $\delta$=7.101ppm, 7.629ppm, 8.187ppm) versus $\alpha$, $\beta$, $\gamma$-methylene protons of poly-$l$-lysine (-$CH_2CH_2CH_2$: $\delta$=1.122ppm, 1.285ppm, 1.553ppm), this indicated that a 100 % of the amines had been reacted. C./D. Dynamic light scattering analysis of PCMs uncross-linked (C) and peptide cross-linked (D). A 50mM PBS buffer solution, at pH 7.4, containing 0.06 mg/ml of PEG-polylysine thiopyridal and 20$\mu$g/ml of ISS DNA was made and filtered through a 200nm syringe filter. This solution was then analyzed by dynamic light scattering (Zetasizer Nano ZS, Malvern Instruments), using the Cumulant method (C). This solution was then crosslinked by adding 0.12 mg of peptide antigen (II), after 3 hours of reaction the solution was analyzed by DLS as described above, the size and the size distribution of the crosslinked micelles are shown in D). E. Crosslinking reaction of cysteines on peptide anigen (II). F. UV analysis of crosslinking reaction between peptide anigen (II) and block copolymer micelles. Block copolymer micelles were formed between I and ISS DNA by mixing 0.5 mg of I with 0.1 mg of ISS DNA (representing a 15/1 amine to phosphate ratio), in 0.5

ml of 50mM $NaH_2PO_4$ buffer (pH 7.4), in an eppendeorf tube. After incubation for 2 hours at room temperature, 0.1mg of II (representing a 1:1 cysteine to thiopyridal ratio) was added to the micelles. The crosslinking reaction between the cysteines on II with the thiopyridal groups in the micelles was determined by UV analysis at 342nm (representing the released thiopyridone). The percent of cysteine groups reacted was determined by the following formula:

$$reacted\ peptide\ (\%) = \frac{ABS_1 - ABS_2}{ABS_o} \times 100\%$$

where: $ABS_1$ = UV absorption at 342nm for the peptide-crosslinked micelles reaction (filled circles); $ABS_2$ = UV absorption at 342nm for the uncrosslinked micelles, without peptide (empty squares); $ABS_o$ = UV absorption at 342nm when all of the thiopyridone groups have been reacted (by addition of DTT) (empty circles).

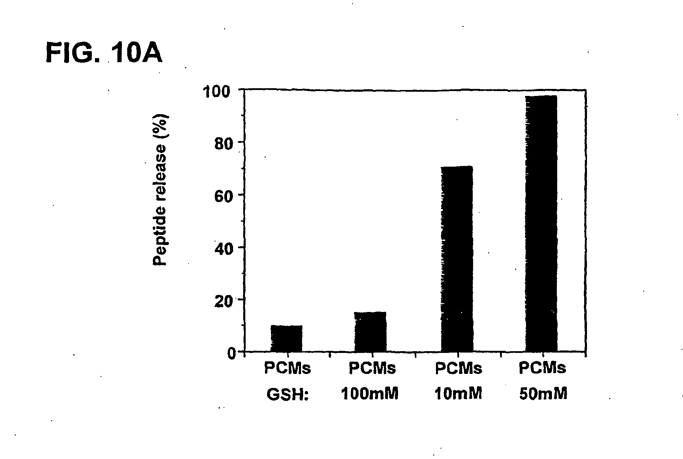

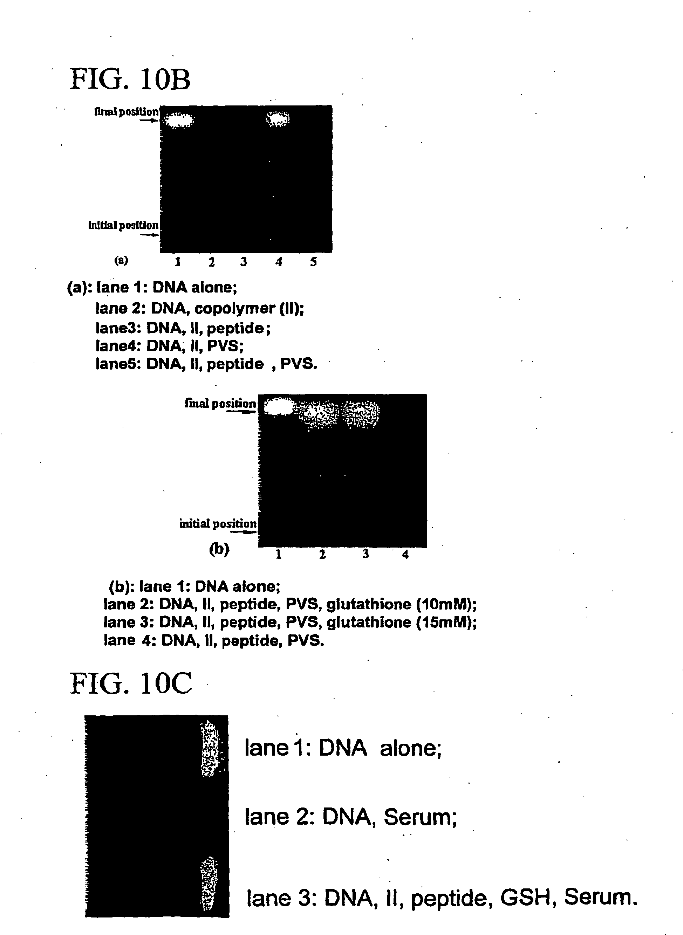

Figure 10 shows the effects of GSH on release of peptides and DNA.A. GSH sensitive peptide release. The stimuli responsive release of peptides from the PCMs due to the presence of GSH was investigated to determine if the PCMs will release peptide antigens after phagocytosis. The PCMs were incubated with different concentrations of GSH, for 24 hours in 50mM pH 7.4 PBS buffer, and then analyzed by HPLC to determine the release of peptides. This figure demonstrates that the release of peptides is triggered by the presence of GSH. Incubation of the PCMs with 10mM GSH (intracellular levels) induces the release of 71% of peptide, whereas incubation of the PCMs with just buffer causes the release of only 10% of peptides. B. GSH sensitive DNA release. The ability of the PCMs to protect encapsulated ISS-DNA from degradation by serum nucleases was investigated. PCMs were synthesized (as described above) and incubated with 10% serum for 12 hours, these PCMs were then examined by gel electrophoresis to determine the stability of the encapsulated ISS-DNA. As a control, ISS-DNA by itself was incubated with serum. This figure demonstrates that ISS-DNA, by itself, is completely hydrolyzed in 10% serum (Lane 2), in contrast ISS-DNA encapsulated in the PCMs is protected from serum nucleases, presumably because of the effects of the crosslinking (Lane 3), which should prevent nucleases from entering the micelle. C. ISS-DNA is protected from serum nucleases in the PCMs. A key advantage of the PCM strategy is that it generates a crosslinked delivery system. This crosslinking should stabilize the PCMs in vivo. The stability of the PCMs to decomposition was investigated by mixing the negatively charged polymer, poly(vinyl sulfate) (PVS) with the PCMs, this mixture was then analyzed by gel electrophoresis to determine the quantity of ISS-DNA displaced by the PVS. As a control, PVS was also incubated with uncrosslinked micelles that were just composed of II and ISS-DNA. Figure A, lane 4, demonstrates that PVS can disrupt uncrosslinked micelles that are just composed of ISS-DNA and II. In contrast, A, lane 5 demonstrates that PVS cannot displace ISS-DNA from the PCMs, presumably because the peptide crosslinking prevents the PVS from diffusing into the micelles and displacing the ISS-DNA. Importantly, after incubation of the PCMs with intracellular concentrations of GSH, the PCMs release encapsulated ISS-DNA, in the presence of PVS, demonstrating that the micelles should release their contents after phagocytosis. (lanes 2 and 3 in b)). Charge ratio of ISS-DNA to I is 1 to 15 (-/+); 1μg of DNA was loaded in each lane; GSH (100μM) was added to lane 3 to induce release of encapsulated ISS-DNA. All samples were incubated with 10% serum at room temperature for 12 h.

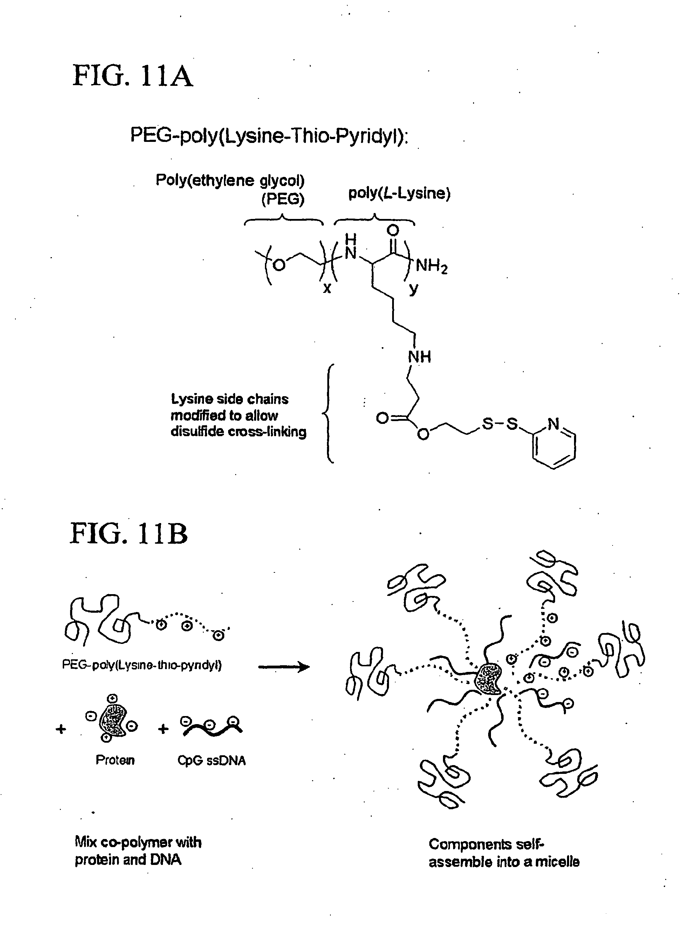

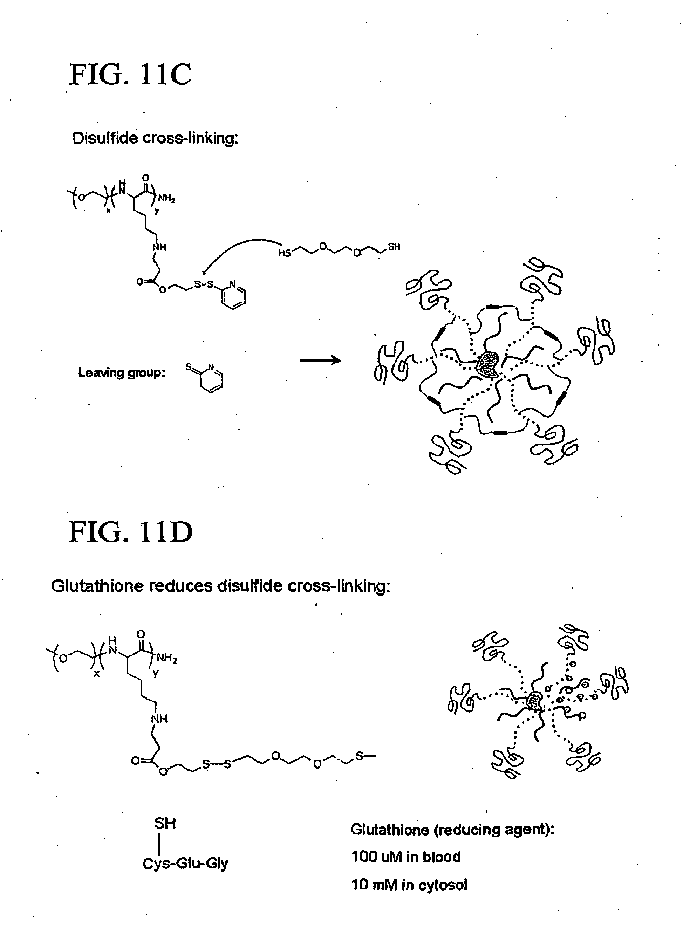

Figure 11 depicts block copolymer micelles: A. Chemical structure of PEG-poly(Lysine-Thio-Pyridyl). PEG chain gives stability to the micelles, Polylysine segment is used for electrostatic interactions with proteins and DNA or RNA. Thiopyridal group is for subsequent crosslinking via a disulfide bond. B. Step I: Mix PEG-poly(Lysine-thiopyridal) with DNA and protein, form micelles, with PEG on the outside. C. Step II: Crosslink micelles with a di-thiol containing molecule, such as di-thioethylene glycol. D. Crosslinked micelles are reduced by Glutathione, which has a much higher concentration in the cell than in the blood.

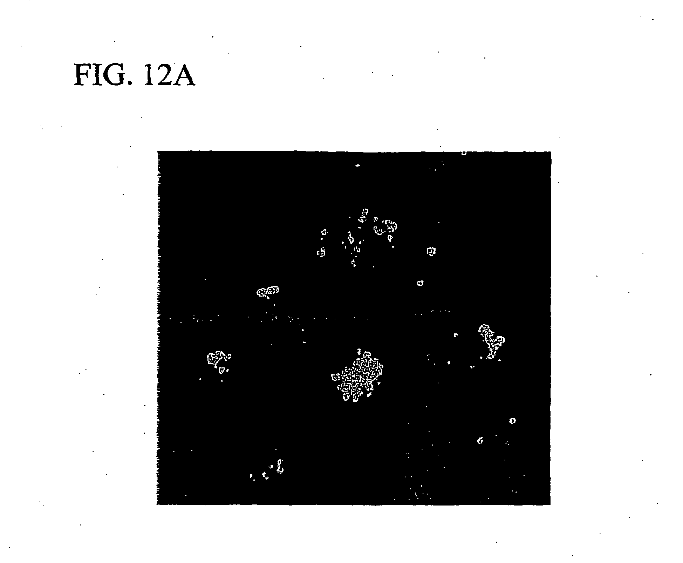

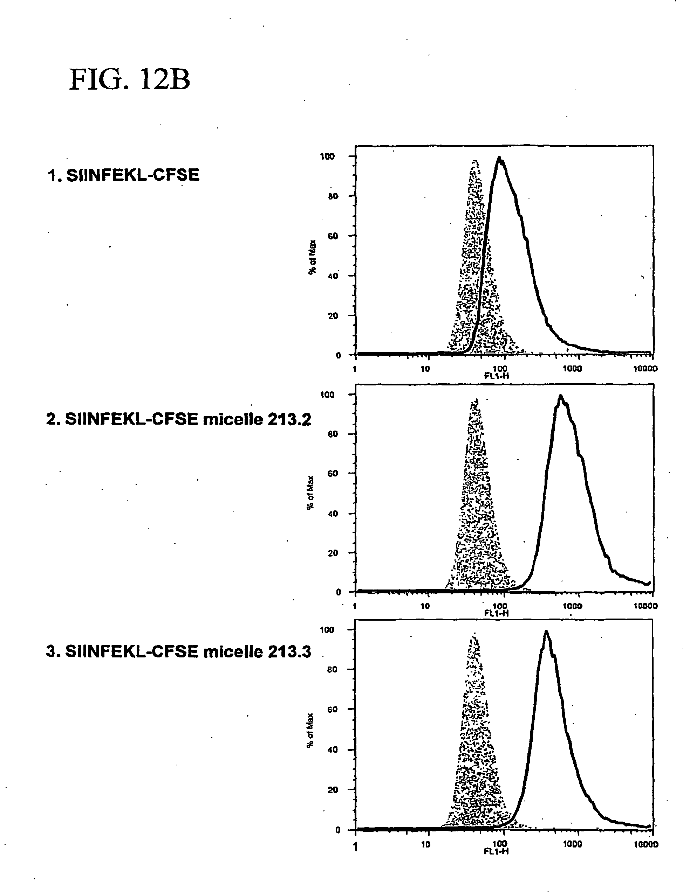

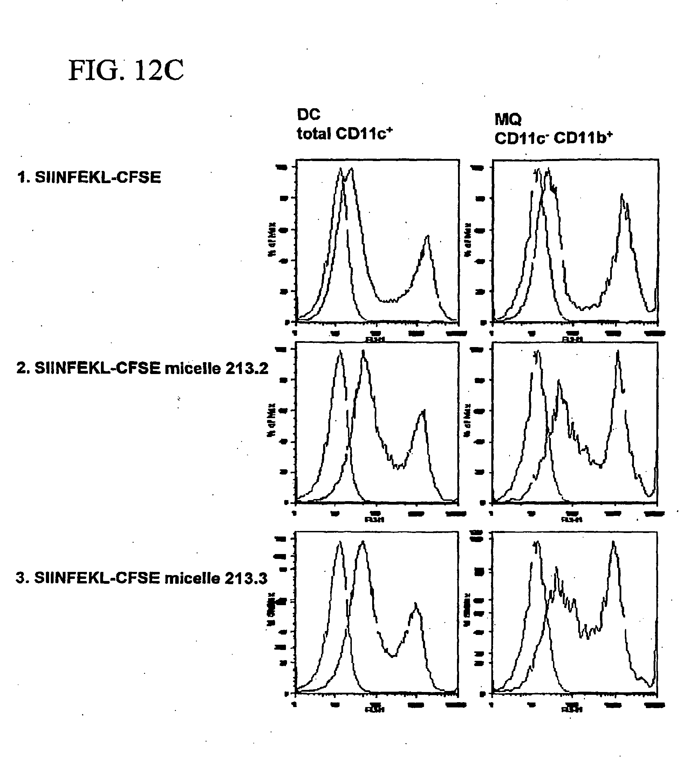

Figure 12 shows the immunology of micelles. A. Uptake of SIINFEKL-CFSE micelles by human monocyte derived DCs. Human PBMC derived DCs (day 6 of culture) were pulsed with SIINFEKL/CFSE micelles for 4h in 37°C at the concentration of 10μg/ml, 5x10*5 cell/well, 96-U wells, RPMI/10% FCS. Cells were washed, fixed on the figures and prepared for confocal microscope imaging. B. Efficient uptake of micelle encapsulated SIINFEKL peptide by human monocyte derived DCs. Human PBMC derived DCs (day 6 of culture) were pulsed with SIINFEKL/CFSE plain (1) or micelle formulated (2,3) for 4h in 37°C at the concentration of 10μg/ml, 5x10*5 cell/well, 96-U wells, RPMI/10% FCS. Cells were washed, stained for CD11c and HLADR. Cells were gated for CD11c+, HLADR+ cells and analysed for CFSE fluorescence. C. Efficient uptake of micelle encapsulated SIINFEKL peptide by mouse DCs and Macrophages. Total mouse C57B1/6J FLT3-L splenocytes were pulsed with 1 or 10 μg/ml of CFSE labeled plain SIINFEKL (1) or micelle formulated peptide (2,3) for 4 hours (blue line), or remained untreated (red line) at 37°C at the concentration 1x10*6 cell/well, 96-U wells, RPMI/10% FCS. Cells were stained for CD11c and CD11b and analyzed for CFSE positive fluorescence.

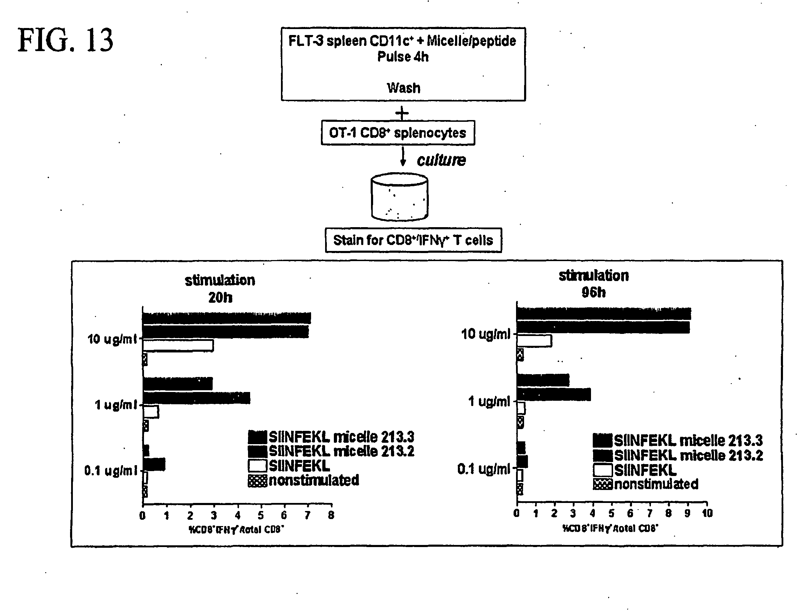

Figure 13 is a bar graph showing that micelle formulated SIINFEKL peptide induces potent T cell responses *in-vitro*. Purified CD11c[+] cells from a syngeneic C57B1/6J H-2[b] FLT3-L treated mouse were pulsed for 4 hours with indicated concentrations and combinations of plain or micelle formulated SIINFEKL peptide. Cells were washed and cocultured with total OT-1 splenocytes for 20h (left panel) or 96h (right panel). Cells were stained for CD8 and intracellular IFN$\gamma$ and analyzed on a flow cytometer. Graphs represent: micelle SIINFEKL 213.3 (black bar), micelle SIINFEKL 213.2 (grey bar), plain SIINFEKL (empty bar), medium (dotted bar) stimulated CD11c+ DCs.

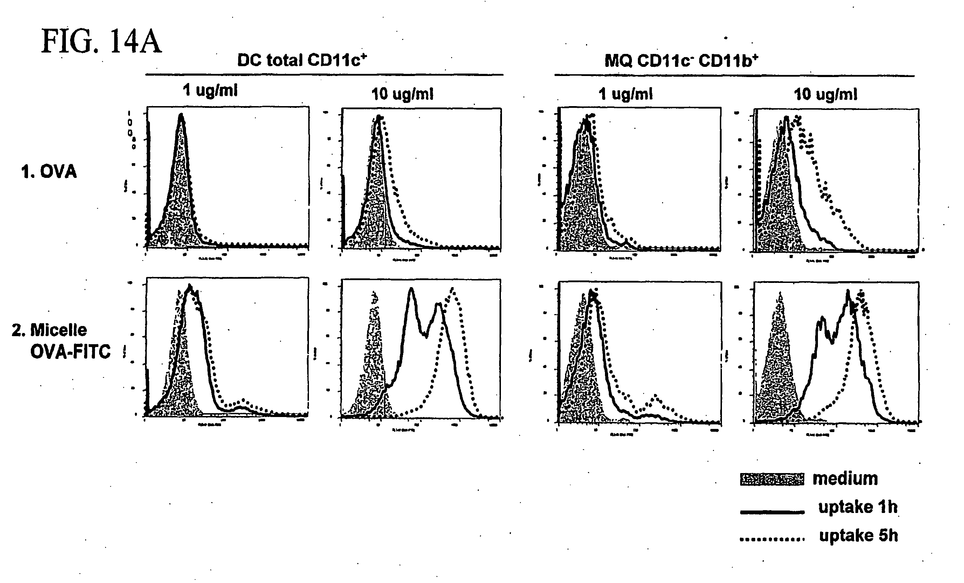

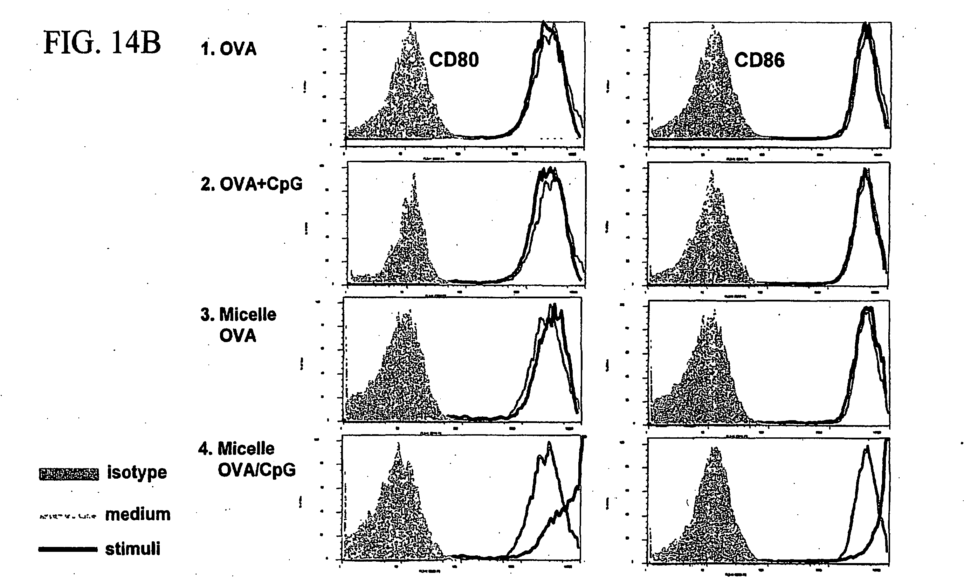

Figure 14 shows the immunology of micelles. A. Efficient uptake of micelle encapsulated OVA protein by mouse DCs and Macrophages. Total mouse C57B1/6J FLT3-L splenocytes were pulsed with 1 or 10 $\mu$g/ml of FITC labeled plain ovalbumin (1) or micelle formulated protein (2) for 1 hour (black line), 5 hours (dotted line) or remained untreated (shaded). 37°C at the concentration 1x10*6 cell/well, 96-U wells, RPMI/10% FCS. Cells were stained for CD11c and CD11b and analyzed for FITC positive fluorescence. B. Micelle encapsulated OVA/CpG activates DCs *in-vitro*. Total mouse C57BI/6J FLT3-L splenocytes were pulsed with 1 $\mu$g/ml of plain OVA (1) plain OVA + 1$\mu$g of CpG (2) or micelle formulated OVA (3) or micelle OVA + 1$\mu$g of CpG (4) for 24 hours. CD11c+ cells were analysed for and CD80 or CD86 markers. Data represent isotype control (shaded), untreated (grey line) or stimuli treated (black line) cells.

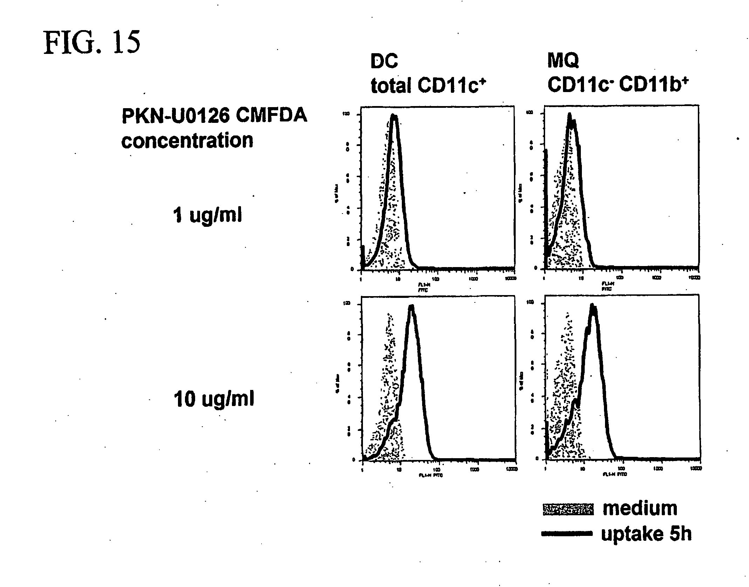

Figure 15 is a graph depicting the immunology of polyketal particles. Uptake of polyketal particle (PKN) encapsulated U0126 by mouse DCs and Macrophages *in-vitro*. Total mouse C57B1/6J FLT3-L splenocytes were pulsed with 1 or 10 $\mu$g/ml of CMFDA labeled Polyketal Particle carrying U0126 ERK inhibitor for 5 hours at 37°C, 1x10*6 cell/well, 96-U wells, RPMI/10% FCS. Cells were stained for CD11c and CD11b and analyzed for CMFDA positive fluorescence. Data represent medium treated cells (shaded line) or 5 hours uptake (black line).

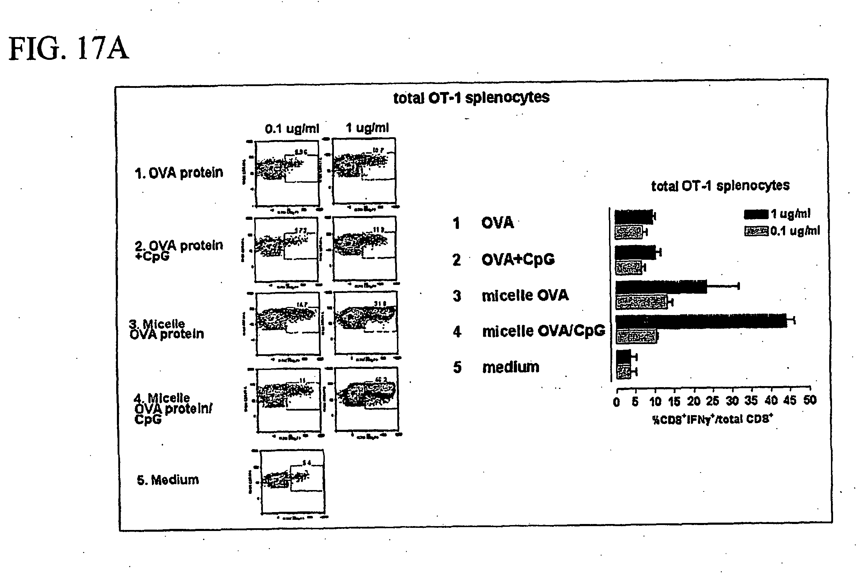

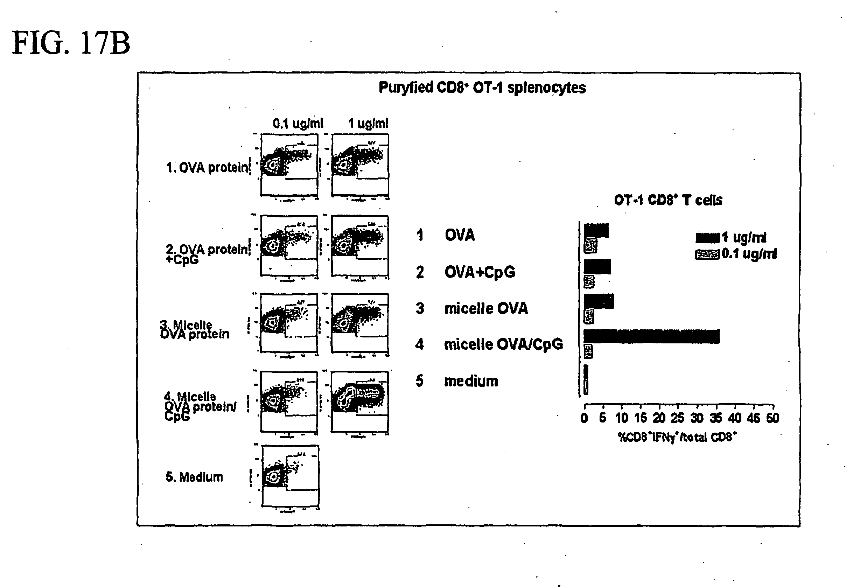

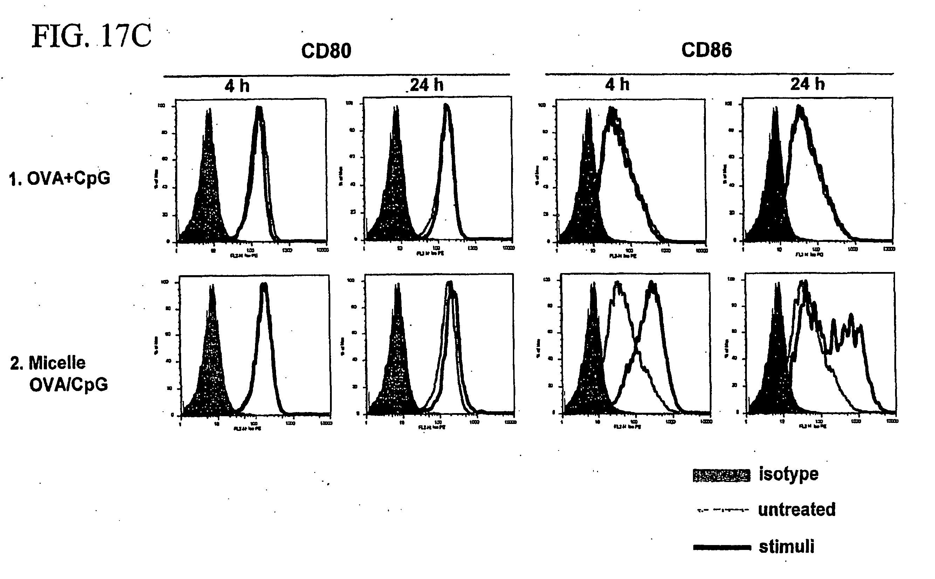

Figure 17 shows the immunology of micelles. A. Micelle formulated antigen induces potent T cell responses *in-vitro*. Purified CD11c[+] cells from a syngeneic C57B1/6J H-2[b] mouse were pulsed for 4 hours with indicated concentrations and combinations of ovalbumin and CpG plain or micelle formulated. Cells were washed and 1x10*5 of CD11c+ were cocultured with 1x10*6 of total OT-1 (SIINFEKL specific) splenocytes for 5 days. After 5 days cells were restimulated with a SIINFEKL peptide (1$\mu$g/ml) with BFA (5ug/ml) for 6 hours and stained for CD8 and intracellular IFN$\gamma$. Left panel represents the Flow Cytometer analysis; right panel shows the summary of the data. B. Micelle formulated antigens overcome CD4+ dependent mechanisms of CD8+ T cells induction. Purified CD11c[+] cells from a syngeneic C57B1/6J H-2[b] mouse were pulsed for 4 hours with indicated concentrations and combinations of ovalbumin and CpG plain or micelle formulated. Cells were washed and 1x10*5 of CD11c were cocultured with 5x10*5 of CD8+ MACS purified OT-1 splenocytes (purity of ~90%) for 5 days. After 5 days cells were restimulated with a SIINFEKL peptide (1$\mu$g/ml) with BFA (5$\mu$g/ml) for 6 hours and stained for CD8 and intracellular IFN$\gamma$. Left panel represents the Flow Cytometer analysis; right panel shows the summary of the data. C. Micelle formulated antigen activate DCs *in-vivo*. C57B1/6J mice (2/group) were injected i.v. with 5 $\mu$g/mouse of ovalbumin/CpG as plain or micelle formulated in 500ul PBS. Spleens were harvested at 4 and 24 hours post injection, treated with collagenase (30min/37C), homogenized, and treated with erythrocyte lysis buffer. CD11c+ cells were stained for CD80 or CD86 markers and analyzed with a flow cytometer. Data represent isotype control (shaded), nontreated mouse (grey line) or antigen injected mouse (black line).

Figure 18 shows the immunology of micelles and polyketal particles. A. Micelle formulated vaccines induce strong T cell responses *in-vivo* - CD8[+] IFN$\gamma$[+]. Cohorts of 4 C57B1/6J mice were vaccinated s.c. with 5 $\mu$g/mouse of OVA (1) or OVA + CpG (2), micelle OVA (3), micelle OVA/CpG (4). Animals were boosted at day 36 (BOOST 1) and 84 (BOOST 2) using the same antigen formulations. Blood was harvested at days 6 post BOOST 2 and PBMCs were isolated using Histopaque gradient method, and restimulated with SIINFEKL peptide (1$\mu$g/ml) and BFA (5$\mu$g/ml) for 6 hours and stained for CD8 and intracellular IFN$\gamma$. Left panel represents the Flow Cytometry analysis and gating strategy for selected mice; right panel shows the summary of the data as a % of CD8[+] IFN$\gamma$[+] cells of total CD8[+] T cells. B. Micelle formulated vaccines induce strong T cell responses *in-vivo* - CD8[+] TNF$\alpha$[+]. Cohorts of 4 C57B1/6J mice were vaccinated s.c. with 5 $\mu$g/mouse of OVA (1) or OVA + CpG (2), micelle OVA (3), micelle OVA/CpG (4) micelle. Animals were boosted at day 36 (BOOST 1) and 84 (BOOST 2) using the same antigen formulations. Blood was harvested at days 6 post BOOST 2 and PBMCs were isolated using Histopaque gradient method. PBMCs from 4 mice were pooled and restimulated with SIINFEKL peptide (1$\mu$g/ml) and BFA (5$\mu$g/ml) for 6 hours and stained for CD8 and intracellular 1NF$\alpha$ and IL-10. Left panel represents the Flow Cytometer analysis and gating strategy for selected mice; right panel shows the summary of the data as a % of CD8[+] TNF$\alpha$[+] cells of total CD8[+] T cells. C.

Kinetics of specific CD8⁺/IFNγ⁺ T cells after OVA/CpG vaccination. Cohorts of 4 C57B1/6J mice were vaccinated s.c. with 5 μg/mouse of OVA + CpG (grey line) and micelle OVA/CpG (blue line). Animals were primed at day 0 and boosted at day 36 (BOOST 1) and 84 (BOOST 2) using the same antigen formulations. Blood was harvested at distinct days post priming and boosts and PBMCs were isolated using Histopaque gradient method. PBMCs from 4 mice were restimulated with SIINFEKL peptide (1μg/ml) and BFA (5μg/ml) for 6 hours and stained for CD8 and intracellular IFNγ. Panels represent the summary of the data as % of CD8⁺ IFNγ⁺ cells of total CD8⁺ T cells including SEM error bars. D. Kinetics of specific CD8⁺/IFNγ⁺ T cells after OVA + UO126 PKN vaccination. Cohorts of 4 C57B1/6J mice were vaccinated s.c. with 5 μg/mouse of OVA (grey line) micelle OVA (blue line) and micelle OVA + 10μg UO126 PKN( red line). Animals were primed at day 0 and boosted at day 36 (BOOST 1) and 84 (BOOST 2) using the same antigen formulations. Blood was harvested at distinct days post priming and boosts and PBMCs were isolated using Histopaque gradient method. PBMCs from 4 mice were restimulated with SIINFEKL peptide (1μg/ml) and BFA (5μg/ml) for 6 hours and stained for CD8 and intracellular IFNγ. Panels represent the summary of the data as % of CD8⁺ IFNγ⁺ cells of total CD8⁺ T cells including SEM error bars. E. Micelle formulated vaccines induce strong antigen specific IgG antibody response *in-vivo*. Cohorts of 4 C57B1/6J mice were vaccinated s.c. with 5 μg/mouse of OVA (1) or OVA + CpG (2), micelle OVA (3), micelle OVA/CpG (4) micelle OVA+ 10ug of PKN U0126 (5) or micelle OVA/CpG + 10μg of PKN U0126 (6). Animals were boosted at days 36 and 84 using the same antigen formulations. Bleedings were performed at week 4 post priming (PRIME), week 6 post 1st boost (BOOST 1) and week 7 post 2nd boost (BOOST 2). Sera from individual mice were pooled and tested for OVA specific IgG total, IgG1, IgG2a and IgG2b antibody reactivity using plate ELISA. Antibody reactivity was measured in 450nm absorbance of serum serial dilution. Data are represented as a reciprocal of specific anti-OVA antibody. F. Micelle formulated vaccines induce antigen specific IgE and IgM antibody response *in-vivo.* Cohorts of 4 C57B1/6J mice were vaccinated s.c. with 5 μg/mouse of OVA (1) or OVA + CpG (2), micelle OVA (3), micelle OVA/CpG (4) micelle OVA+ 10ug of PKN U0126 (5) or micelle OVA/CpG + 10ug of PKN U0126 (6). Animals were boosted at days 36 and 84 using the same antigen formulations. Bleeding was performed and week 7 post 2nd boost. Sera from individual mice were pooled and tested for OVA specific IgE and IgM antibody reactivity using plate ELISA. Antibody reactivity was measured in 450nm absorbance of serum serial dilution. Data are represented as a reciprocal of specific anti-OVA antibody.

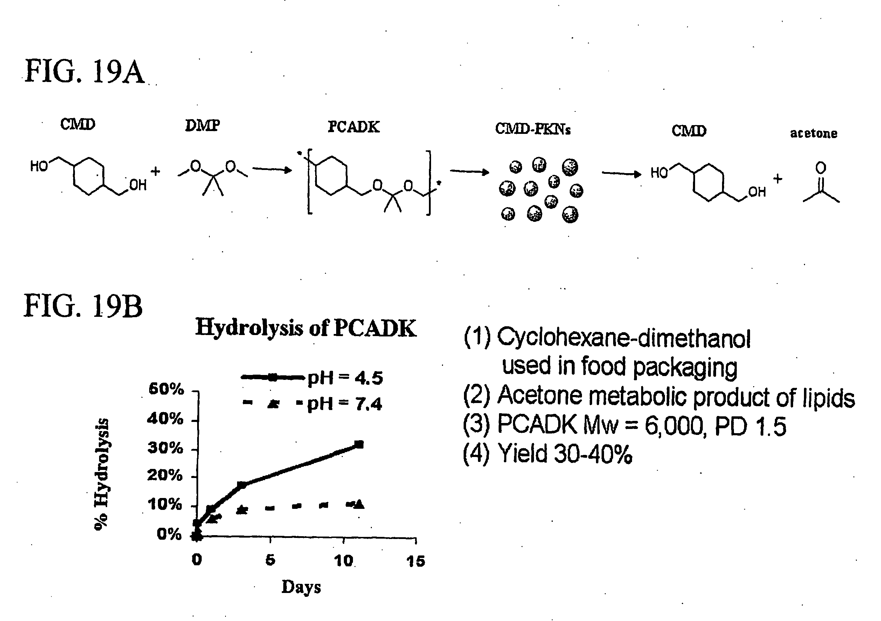

Figure 19 shows polyketals from cyclohexane dimethanol A. Polyketals from cyclohexane dimethanol (termed PCADK) degrade into cyclohexane dimethanol and acetone, both have FDA approval for human use. B. PCADK degrades in an acid sensitive manner. The ketal linkages in PCADK hydrolyze on the order of weeks under physiologic pH conditions. The hydrolysis of the ketal linkages in PCADK were measured by H-NMR at the pHs of 4.5 and 7.4. At the phagosomal pH of 4.5, the ketal linkages of PCADK are approximately. 30% hydrolyzed after 10 days. Based on this result, we anticipate that CAT-PKNs should be completely hydrolyzed, within 4-5 weeks after phagocytosis by macrophages.

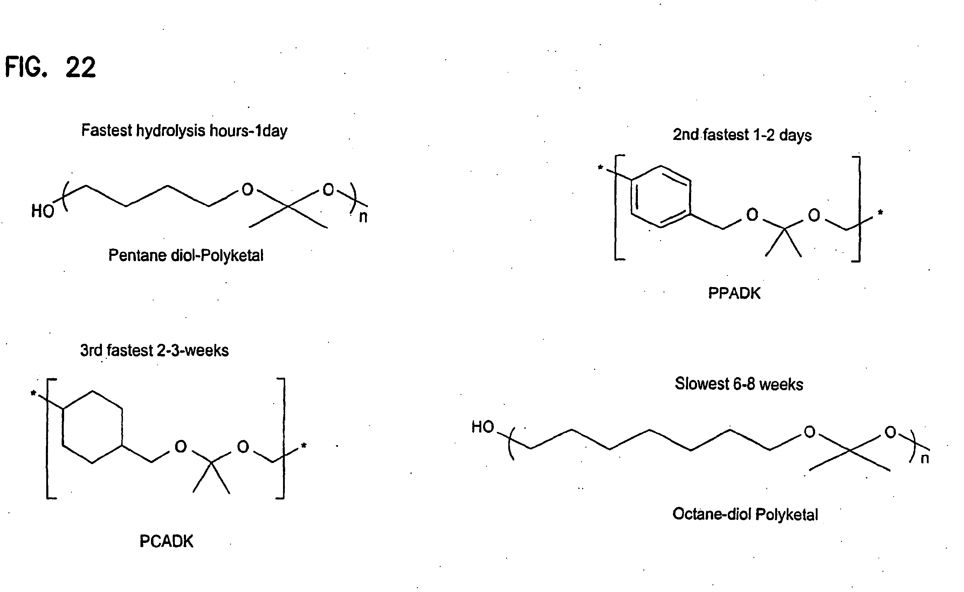

Figure 22 is a chemical representation showing that polyketals with almost any aliphatic diol can be made. The hydrophobicity of the polyketal determines its hydrolysis kinetics.

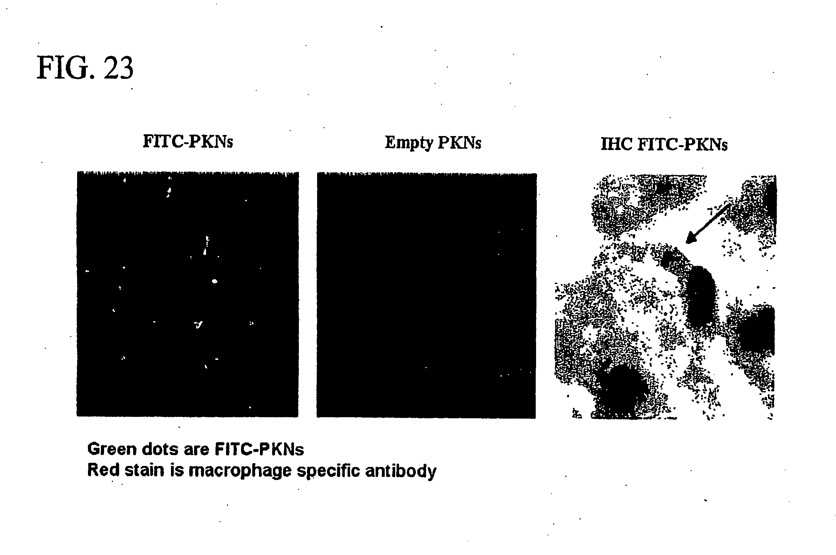

Figure 23 is a photograph showing that FITC labeled polyketals are phagocytosed by liver macrophages. Phagocytosis of PKNs in vivo by Kupffer cells. Mice were injected with either FITC-PKNs or Empty PKNs. The livers of these mice were analyzed by histology. Left: FITC-PKNs are abundantly present in Kupffer cells, as evidenced by the punctate green fluorescence (100x magnification). Middle: empty PKNs generate very little background green fluorescence (100x magnification). Right: immunohistochemistry (IHC) for FITC (red) confirms uptake by Kupffer cells (400x magnification).

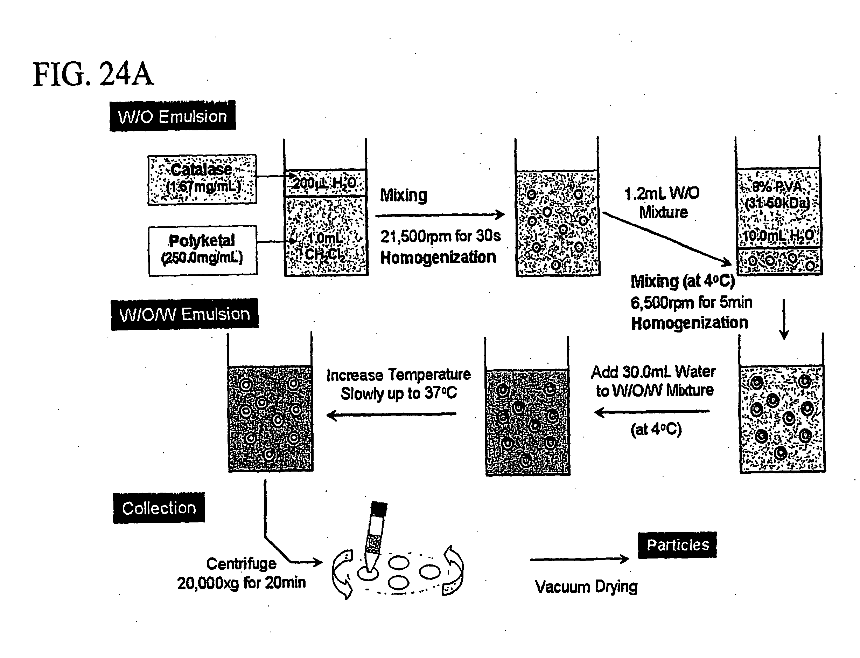

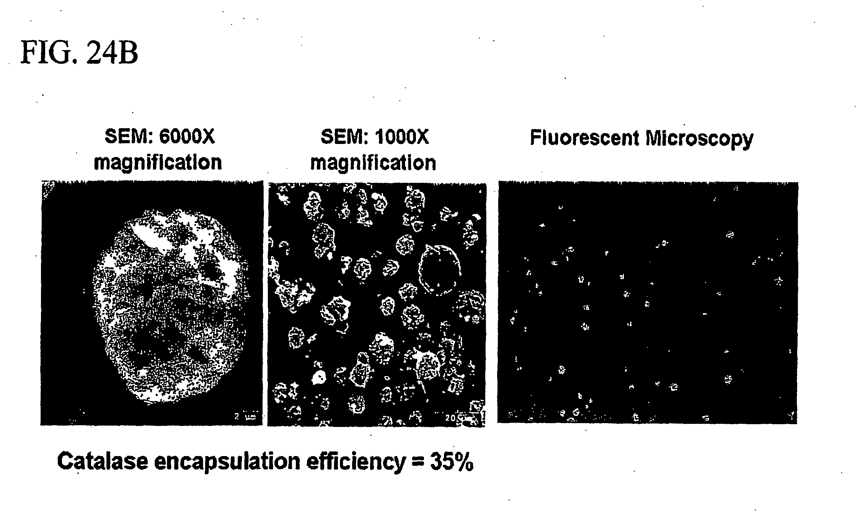

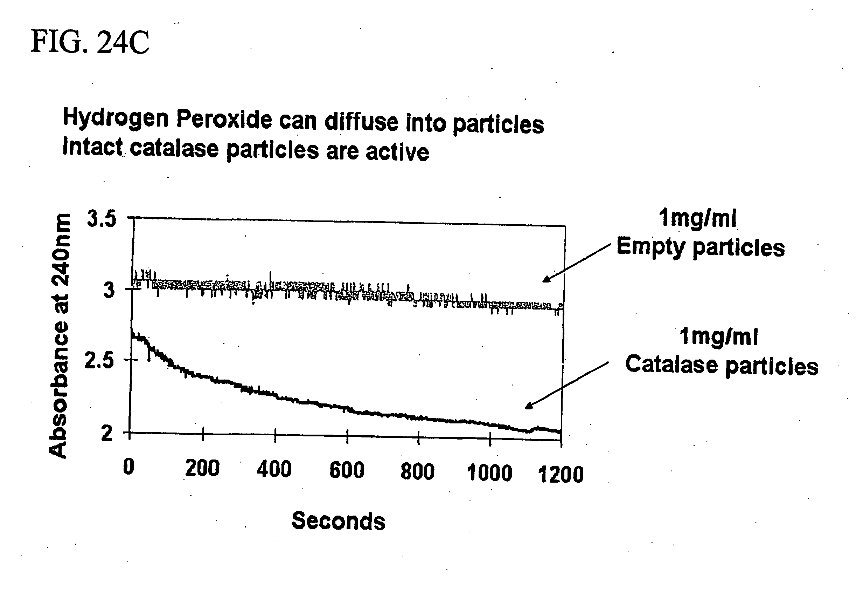

Figure 24. A. Double emulsion procedure used to encapsulate catalase and super oxide dismutase in polyketal particles. A 50 μL aqueous solution of catalase (1 mg/ml) was dispersed into an organic phase, consisting of 75 mg of PCADK, dissolved in 1mL of dichloromethane, using a homogenizer, generating a water in oil (w/o) emulsion. This w/o emulsion was then dripped into 25mL of a 4% PVA solution, which was mechanically stirred with a homogenizer. The resulting w/o/w emulsion was then poured into 225mL of a 4% PVA solution and mechanically stirred for several hours until the methylene chloride evaporated. The resulting particles were isolated by centrifugation, freeze-dried and examined by SEM (B) The protein encapsulation efficiency was 35%. The CAT-PKNs have an average diameter of approximately 8 microns. B. SEM image of catalase containing particles, and fluorescent microscope images of catalase containing particles. C. Catalase particles have enzymatic activity, as evidenced by their ability to decrease the absorbance at 240nm of hydrogen peroxide.

**EP 1 893 661 B1**

**Claims**

1. A biodegradable hydrophobic polyketal polymer comprising ketal groups, wherein each ketal group of the polymer has two oxygen atoms within the polymer backbone.

2. A biodegradable particle comprising the polyketal polymer of claim 1.

3. The particle of claim 2, further comprising one or more active agents.

4. The polymer of claim 1, wherein a ketal group is a 2, 2-dioxypropyl group.

5. The polymer of claim 1, wherein a ketal group is attached to a group selected from the group consisting of alkyl, aryl and cycloalkyl group or is attached to a 1,4-dimethylbenzene group or a 1,4-dimethylcyclohexyl group.

6. The polymer of claim 1, wherein the polymer is poly(1,4-phenylene-acetone dimethylene ketal) or poly(1,4-cyclohexane-acetone dimethylene ketal).

7. The particle of claim 2, wherein the particle is a nanoparticle or microparticle.

8. The particle of claim 7, wherein the particle is from about 50 - 1000 nm in size or from about 200 - 600 nm in size.

9. The particle of claim 3, wherein said active agent is a therapeutic, prophylactic or diagnostic agent.

10. The particle of claim 9, wherein said therapeutic agent is an immunomodulatory agent.

11. The particle of claim 14, wherein said immunomodulatory agent is selected from a group consisting of

    a. a ligand for any of TLR 2, 3, 4, 5, 7, 8, 9, 10 and 1 or combination thereof, and
    b. an inhibitor of a regulatory pathway within dendritic cells, macrophages or antigen-presenting cells.
    c. a ligand for RIG-1, any C-type lectins including dectin-1 and DC-SIGN, or Caterpillar proteins.

12. The particle of claim 11, wherein said inhibitor is selected from the group consisting of inhibitors of (a) ERK, c-Fos, Foxp3, PI3 kinase, JNK, p38, NF-Kb, STAT 1, STAT2, IRF3, IRF7, IFN-alpha signaling; or (b) a SOCS 1, 2, 3, or other SOCS protein.

13. A method for producing and encapsulating the particle of claim 3 comprising the steps of

    a. forming a hydrophobic polymer of a ketal and a diol or an unsaturated alcohol; and
    b. forming a particle of the polymer of a) in the presence of one or more active agents

thereby producing and encapsulating the particle of claim 3.

14. The method of claim 13, wherein the ketal is 2,2-dimethoxypropane.

15. The method of claim 13, wherein the diol is selected from the group consisting of alkyl, aryl and cycloalkyl diols or the diol is 1,4-benzenedimethanol or 1,4-cyclohexanedimethanol.

16. The method of claim 13, wherein the hydrophobic polymer of a) is poly(1,4-phenylene-acetone dimethylene ketal) or poly(1,4-cyclohexane-acetone dimethylene ketal).

17. The method of claim 13, wherein the active agent is a therapeutic, prophylactic or diagnostic agent.

18. The method of claim 17, wherein said therapeutic agent is an immunomodulatory agent.

19. The method of claim 18, wherein said immunomodulatory agent is selected from a group consisting of

    a. a ligand for any of TLR 2, 3, 4, 5, 7, 8, 9, 10 and 11 or combination thereof,
    b. an inhibitor of a regulatory pathway within dendritic cells, macrophages or antigen-presenting cells,

c. a ligand for RIG-1. any C-type lectins such as dectin-1 and DC-SIGN, or any Caterpillar proteins.

20. The method of claim 19, wherein said inhibitor is (a) selected from the group consisting of inhibitors of ERK, c-Fos, Foxp3, PI3 kinase, Akt, JNK, p38, NF-Kb, STAT I, STAT2, IRF3, IRF7, IFN-alpha signaling; or (b) a SOCS 1, 2, or 3 or any SOCS protein.

21. A biodegradable particle produced by the method of claim 13.

22. The use of the particle of claim 3 for the manufacture of a medicament for delivering active agents to a subject, wherein said particle of claim 3 is to be administered to said subject, said particle being degraded in the subject so that the active agent therein is released and delivered to the subject.

23. The use according to claim 22, wherein said medicament is for treating a subject suffering from a disease or disorder.

24. The use of claim 23, wherein the disease or disorder is selected from the group consisting of autoimmune diseases, allergic diseases, infectious diseases, diabetes and cancer.

25. The use of claim 24, wherein the infectious disease is selected from the group consisting of HIV, malaria, TB, SARS, anthrax, Ebola, influenza, avian influenza and HCV.

26. The use of claim 24, wherein the autoimmune disease is selected from the group consisting of lupus, rheumatoid arthritis, psoriasis, asthma and COPD.

27. A pharmaceutical composition comprising the particle of claim 3.

28. The particle of claim 3, wherein said active agent is a protein, peptide, nucleic acid or small molecule.

29. The particle of claim 28, wherein said nucleic acid is siRNA.

**Patentansprüche**

1. Biologisch abbaubares hydrophobes Polyketalpolymer, umfassend Ketalgruppen, wobei jede Ketalgruppe des Polymers zwei Sauerstoffatome in der Polymerhauptkette aufweist.

2. Biologisch abbaubares Teilchen, umfassend das Polyketalpolymer nach Anspruch 1.

3. Teilchen nach Anspruch 2, ferner umfassend einen oder mehrere Wirkstoffe.

4. Polymer nach Anspruch 1, wobei eine Ketalgruppe eine 2,2-Dioxypropylgruppe ist.

5. Polymer nach Anspruch 1, wobei eine Ketalgruppe an eine Gruppe, ausgewählt aus der Gruppe, bestehend aus einer Alkyl-, Aryl- und Cycloalkylgruppe, gebunden ist oder an eine 1,4-Dimethylbenzolgruppe oder eine 1,4-Dimethylcyclohexylgruppe gebunden ist.

6. Polymer nach Anspruch 1, wobei das Polymer Poly(1,4-phenylen-aceton-dimethylen-ketal) oder Poly(1,4-cyclohexan-aceton-dimethylen-ketal) ist.

7. Teilchen nach Anspruch 2, wobei das Teilchen ein Nanoteilchen oder Mikroteilchen ist.

8. Teilchen nach Anspruch 7, wobei das Teilchen eine Größe von etwa 50 - 1000 nm oder etwa 200 - 600 nm hat.

9. Teilchen nach Anspruch 3, wobei der Wirkstoff ein therapeutisches, prophylaktisches oder diagnostisches Mittel ist.

10. Teilchen nach Anspruch 9, wobei das therapeutische Mittel ein immunmodulatorisches Mittel ist.

11. Teilchen nach Anspruch 10, wobei das immunmodulatorische Mittel ausgewählt ist aus einer Gruppe, bestehend aus

a. einem Liganden für einen von TLR 2, 3, 4, 5, 7, 8, 9, 10 und 11 oder eine Kombination davon und
b. einem Inhibitor eines regulatorischen Stoffwechselweges in dendritischen Zellen, Makrophagen oder Antigen-präsentierenden Zellen,
c. einem Liganden für RIG-1, eines der Lektine vom C-Typ, einschließlich Dectin-1 und DC-SIGN, oder Caterpillar-Proteine.

12. Teilchen nach Anspruch 11, wobei der Inhibitor ausgewählt ist aus der Gruppe, bestehend aus Inhibitoren von (a) ERK-, c-Fos-, Foxp3-, PI3-Kinase-, JNK-, p38-, NF-Kb-, STAT 1-, STAT2-, IRF3-, IRF7-, IFN-alpha-Signalstoffen oder (b) einem SOCS 1-, 2-, 3- oder einem anderen SOCS-Protein.

13. Verfahren zur Herstellung und Einkapselung des Teilchens nach Anspruch 3, umfassend die Schritte

a. Bilden eines hydrophoben Polymers aus einem Ketal und einem Diol oder einem ungesättigten Alkohol und
b. Bilden eines Teilchens aus dem Polymer von a) in Gegenwart von einem oder mehreren Wirkstoffen,

wobei das Teilchen nach Anspruch 3 hergestellt und eingekapselt wird.

14. Verfahren nach Anspruch 13, wobei das Ketal 2,2-Dimethoxypropan ist.

15. Verfahren nach Anspruch 13, wobei das Diol ausgewählt ist aus der Gruppe, bestehend aus Alkyl-, Aryl- und Cycloalkyldiolen oder das Diol 1,4-Benzoldimethanol oder 1,4-Cyclohexandimethanol ist.

16. Verfahren nach Anspruch 13, wobei das hydrophobe Polymer von a) Poly(1,4-phenylenaceton-dimethylenketal) oder Poly(1,4-cyclohexan-aceton-dimethylenketal) ist.

17. Verfahren nach Anspruch 13, wobei der Wirkstoff ein therapeutisches, prophylaktisches oder diagnostisches Mittel ist.

18. Verfahren nach Anspruch 17, wobei das therapeutische Mittel ein immunmodulatorisches Mittel ist.

19. Verfahren nach Anspruch 18, wobei das immunmodulatorische Mittel ausgewählt ist aus einer Gruppe, bestehend aus

a. einem Liganden für einen von TLR 2, 3, 4, 5, 7, 8, 9, 10 und 11 oder eine Kombination davon und
b. einem Inhibitor eines regulatorischen Stoffwechselweges in dendritischen Zellen, Makrophagen oder Antigen-präsentierenden Zellen,
c. einem Liganden für RIG-1, eines der Lektine vom C-Typ, einschließlich Dectin-1 und DC-SIGN, oder Caterpillar-Proteine.

20. Verfahren nach Anspruch 19, wobei der Inhibitor (a) ausgewählt ist aus der Gruppe, bestehend aus Inhibitoren von ERK-, c-Fos-, Foxp3-, PI3-Kinase-, Akt-, JNK-, p38-, NF-Kb-, STAT 1-, STAT2-, IRF3-, IRF7-, IFN-alpha-Signalstoffen, oder (b) ein SOCS 1-, 2-, 3- oder ein anderes SOCS-Protein ist.

21. Biologisch abbaubares Teilchen, hergestellt durch das Verfahren nach Anspruch 13.

22. Verwendung des Teilchens nach Anspruch 3 zur Herstellung eines Medikaments zur Gabe von Wirkstoffen an ein Individuum, wobei das Teilchen nach Anspruch 3 an das Individuum verabreicht werden soll, wobei das Teilchen in dem Individuum so abgebaut wird, dass der Wirkstoff darin freigesetzt und an das Individuum abgegeben wird.

23. Verwendung nach Anspruch 22, wobei das Medikament zur Behandlung eines Individuums, das an einer Krankheit oder Störung leidet, vorgesehen ist.

24. Verwendung nach Anspruch 23, wobei die Krankheit oder Störung ausgewählt ist aus der Gruppe, bestehend aus Autoimmunerkrankungen, allergischen Erkrankungen, Infektionskrankheiten, Diabetes und Krebs.

25. Verwendung nach Anspruch 24, wobei die Infektionskrankheit ausgewählt ist aus der Gruppe, bestehend aus HIV, Malaria, TB, SARS, Milzbrand, Ebolafieber, Grippe, Vogelgrippe und HCV.

**26.** Verwendung nach Anspruch 24, wobei die Autoimmunerkrankung ausgewählt ist aus der Gruppe, bestehend aus Lupus, Rheumatoidarthritis, Psoriasis, Asthma und COPD.

**27.** Pharmazeutische Zusammensetzung, umfassend das Teilchen nach Anspruch 3.

**28.** Teilchen nach Anspruch 3, wobei der Wirkstoff ein Protein, ein Peptid, eine Nukleinsäure oder ein kleines Molekül ist.

**29.** Teilchen nach Anspruch 28, wobei die Nukleinsäure siRNA ist.

**Revendications**

**1.** Polymère de polycétal biodégradable hydrophobe comprenant des groupements cétal, dans lequel chaque groupement cétal du polymère présente deux atomes d'oxygène dans l'ossature du polymère.

**2.** Particule biodégradable comprenant le polymère de polycétal selon la revendication 1.

**3.** Particule selon la revendication 2, comprenant en outre un ou plusieurs agents actifs.

**4.** Polymère selon la revendication 1, dans lequel un groupement cétal est un groupement 2,2-dioxypropyle.

**5.** Polymère selon la revendication 1, dans lequel un groupement cétal est relié à un groupement choisi dans le groupe constitué des groupements alkyle, aryle et cycloalkyle ou est relié à un groupement 1,4-diméthylbenzène ou 1,4-diméthylcyclohexyle.

**6.** Polymère selon la revendication 1, dans lequel le polymère est le poly(diméthylène-cétal de 1,4-phénylène-acétone) ou le poly(diméthylène-cétal de 1,4-cyclohexane-acétone).

**7.** Particule selon la revendication 2, dans laquelle la particule est une nanoparticule ou une microparticule.

**8.** Particule selon la revendication 7, dans laquelle la particule présente une taille d'environ 50 à 1000 nm ou d'environ 200 à 600 nm.

**9.** Particule selon la revendication 3, dans laquelle ledit agent actif est un agent thérapeutique, prophylactique ou diagnostique.

**10.** Particule selon la revendication 9, dans laquelle ledit agent thérapeutique est un agent immunomodulateur.

**11.** Particule selon la revendication 10, dans laquelle ledit agent immunomodulateur est choisi dans le groupe constitué des composés suivants :

a. un ligand pour l'un quelconque des TLR de type 2, 3, 4, 5, 7, 8, 9, 10 et 11 ou une combinaison de ceux-ci, et
b. un inhibiteur d'une voie de régulation dans des cellules dendritiques, des macrophages ou des cellules présentatrices d'antigène,
c. un ligand pour le RIG-1, une lectine quelconque de type C, notamment la dectine-1 et la DC-SIGN, ou des protéines caterpillar.

**12.** Particule selon la revendication 11, dans laquelle ledit inhibiteur est choisi dans le groupe constitué des inhibiteurs (a) de la signalisation par ERK, c-Fos, Foxp3, PI3 kinase, JNK, p38, NF-Kb, STAT1, STAT2, IRF3, IRF7, IFN-alpha ; ou (b) d'une protéine SOCS 1, 2, 3 ou d'une autre protéine de type SOCS.

**13.** Procédé pour la production et l'encapsulation de la particule selon la revendication 3, comprenant les étapes consistant à :

a. former un polymère hydrophobe à base d'un cétal et d'un diol ou d'un alcool insaturé ; et
b. former une particule du polymère obtenu au point a) en présence d'un ou de plusieurs agents actifs de manière à produire et à encapsuler la particule selon la revendication 3.

**EP 1 893 661 B1**

14. Procédé selon la revendication 13, dans lequel le cétal est le 2,2-diméthoxypropane.

15. Procédé selon la revendication 13, dans lequel le diol est choisi dans le groupe constitué d' alkyldiols, d'aryldiols et de cycloalkyldiols ou le diol est le 1,4-benzènediméthanol ou le 1,4-cyclohexanediméthanol.

16. Procédé selon la revendication 13, dans lequel le polymère hydrophobe obtenu au point a) est le poly(diméthylène-cétal de 1,4-phénylène-acétone) ou le poly(diméthylène-cétal de 1,4-cyclohexane-acétone).

17. Procédé selon la revendication 13, dans lequel l'agent actif est un agent thérapeutique, prophylactique ou diagnostique.

18. Procédé selon la revendication 17, dans lequel ledit agent thérapeutique est un agent immunomodulateur.

19. Procédé selon la revendication 18, dans lequel ledit agent immunomodulateur est choisi dans le groupe constitué des composés suivants :

> a. un ligand pour l'un quelconque des TLR de type 2, 3, 4, 5, 7, 8, 9, 10 et 11 ou une combinaison de ceux-ci,
> b. un inhibiteur d'une voie de régulation dans des cellules dendritiques, des macrophages ou des cellules présentatrices d'antigène,
> c. un ligand pour le RIG-1, une lectine quelconque de type C, notamment la dectine-1 et la DC-SIGN, ou des protéines caterpillar.

20. Procédé selon la revendication 19, dans lequel ledit inhibiteur est (a) choisi dans le groupe constitué des inhibiteurs de la signalisation par ERK, c-Fos, Foxp3, PI3 kinase, Akt, JNK, p38, NF-Kb, STAT1, STAT2, IRF3, IRF7, IFN-alpha ; ou (b) d'une protéine SOCS 1, 2 ou 3 ou d'une protéine SOCS quelconque.

21. Particule biodégradable produite par le procédé selon la revendication 13.

22. Utilisation de la particule selon la revendication 3 pour la fabrication d'un médicament destiné à l'administration d'agents actifs à un sujet, dans laquelle ladite particule selon la revendication 3 doit être administrée audit sujet, ladite particule étant dégradée dans le corps du sujet de sorte que l'agent actif qui y est contenu soit libéré et délivré au sujet.

23. Utilisation selon la revendication 22, dans laquelle ledit médicament vise à traiter un sujet souffrant d'une maladie ou d'un trouble.

24. Utilisation selon la revendication 23, dans laquelle la maladie ou le trouble est choisi dans le groupe constitué des maladies auto-immunes, des maladies allergiques, des maladies infectieuses, du diabète et du cancer.

25. Utilisation selon la revendication 24, dans laquelle la maladie infectieuse est choisie dans le groupe constitué du VIH, de la malaria, de la tuberculose (TB), du SARS, de l'anthrax, du virus Ebola, de la grippe, de la grippe aviaire et du VHC.

26. Utilisation selon la revendication 24, dans laquelle la maladie auto-immune est choisie dans le groupe constitué du lupus, de la polyarthrite rhumatoïde, du psoriasis, de l'asthme et de la BPCO.

27. Composition pharmaceutique comprenant la particule selon la revendication 3.

28. Particule selon la revendication 3, dans laquelle ledit agent actif est une protéine, un peptide, un acide nucléique ou une petite molécule.

29. Particule selon la revendication 28, dans laquelle ledit acide nucléique est un petit ARN interférent (pARNi).

**24**

## FIG. 1

## FIG. 2

(A) HO—⟨CH₂⟩—CH₂OH  +  CH₃O—C(CH₃)₂—OCH₃  ⇌(H₃O⁺, −MeOH (distilled off))  HO—CH₂—⟨⟩—CH₂—O—C(CH₃)₂—OCH₃

1,4-benzenedimethanol    2,2-dimethoxypropane                                                                    **1**

(B) HO—CH₂—⟨⟩—CH₂—O—C(CH₃)₂—OCH₃  +  HO—CH₂—⟨⟩—CH₂—O—C(CH₃)₂—OCH₃  ⇌(H₃O⁺, −MeOH (distilled off))  *⟨⟩—CH₂—O—C(CH₃)₂—O⟩*

           **1**                              **1**                                                                           **2**

(C) *⟨⟩—CH₂—O—C(CH₃)₂—O⟩*  →(Particle Formation)  200 - 600 nm  →(H₃O⁺)  HO—CH₂—⟨⟩—CH₂OH  +  acetone

           **2**                                                                        1,4-benzenedimethanol

EP 1 893 661 B1

## FIG. 3A

## FIG. 3B

# FIG. 4

FIG. 5

EP 1 893 661 B1

# FIG. 6A

Step 1. Combine "oil phase" and "water phase".

*Oil Phase*

*Water Phase*

+

Polyketal + Drug in CHCl$_3$     Polyvinyl alcohol (PVA) emulsifier in H$_2$O

# FIG. 6B

Step 2. Sonicate to form microscopic droplets.

Step 3. Evaporate CHCl$_3$ to encapsulate drug in particles.

Droplet stabilized by emulsifier

Microscopic emulsion

Particle suspension

FIG. 7

Fluorescein

PKN

EP 1 893 661 B1

# FIG. 8

Uncrosslinked micelle

Peptide crosslinked micelle

ISS DNA

PEG

Micelle formation

Crosslinked by II

Phagocytosis by APCs

Cleavage by GSH

HS SH
HS SH

(I)
PEG-PLL-thiopyridal

HS SH
HS SH

(II)
Peptide antigen

Release of II and ISS DNA

**FIG. 9A**

FIG. 9B

EP 1 893 661 B1

## FIG. 9C

Size Distribution by Intensity

Z-Average size (nm): 57.37

Polydispersity index: 0.267

## FIG. 9D

Size Distribution by Intensity

Z-Average size (nm): 50.91

Polydispersity index: 0.164

## FIG. 9E

## FIG. 9F

FIG. 10A

# FIG. 10B

(a): lane 1: DNA alone;
   lane 2: DNA, copolymer (II);
   lane3: DNA, II, peptide;
   lane4: DNA, II, PVS;
   lane5: DNA, II, peptide , PVS.

(b): lane 1: DNA alone;
   lane 2: DNA, II, peptide, PVS, glutathione (10mM);
   lane 3: DNA, II, peptide, PVS, glutathione (15mM);
   lane 4: DNA, II, peptide, PVS.

# FIG. 10C

lane 1: DNA  alone;

lane 2: DNA, Serum;

lane 3: DNA, II, peptide, GSH, Serum.

# FIG. 11A

PEG-poly(Lysine-Thio-Pyridyl):

Poly(ethylene glycol) (PEG)

poly(L-Lysine)

Lysine side chains modified to allow disulfide cross-linking

# FIG. 11B

PEG-poly(Lysine-thio-pyridyl)

+ Protein + CpG ssDNA

Mix co-polymer with protein and DNA

Components self-assemble into a micelle

# FIG. 11C

Disulfide cross-linking:

Leaving group:

# FIG. 11D

Glutathione reduces disulfide cross-linking:

SH
|
Cys-Glu-Gly

Glutathione (reducing agent):

100 uM in blood

10 mM in cytosol

FIG. 12A

## FIG. 12B

**1. SIINFEKL-CFSE**

**2. SIINFEKL-CFSE micelle 213.2**

**3. SIINFEKL-CFSE micelle 213.3**

# FIG. 12C

|  | DC<br>total CD11c<sup>+</sup> | MQ<br>CD11c<sup>-</sup> CD11b<sup>+</sup> |
|---|---|---|

**1. SIINFEKL-CFSE**

**2. SIINFEKL-CFSE micelle 213.2**

**3. SIINFEKL-CFSE micelle 213.3**

# FIG. 13

| FLT-3 spleen CD11c⁺ + Micelle/peptide |
| Pulse 4h |
| Wash |

$+$

OT-1 CD8⁺ splenocytes

↓ *culture*

Stain for CD8⁺IFNγ⁺ T cells

stimulation 20h

| 10 ug/ml |
| 1 ug/ml |
| 0.1 ug/ml |

■ SIINFEKL micelle 213.3
■ SIINFEKL micelle 213.2
□ SIINFEKL
▨ nonstimulated

0 1 2 3 4 5 6 7 8
%CD8⁺IFNγ⁺/total CD8⁺

stimulation 96h

| 10 ug/ml |
| 1 ug/ml |
| 0.1 ug/ml |

■ SIINFEKL micelle 213.3
■ SIINFEKL micelle 213.2
□ SIINFEKL
▨ nonstimulated

0 1 2 3 4 5 6 7 8 9 10
%CD8⁺IFNγ⁺/total CD8⁺

# FIG. 14A

**DC total CD11c⁺**

**MQ CD11c⁻ CD11b⁺**

| 1 ug/ml | 10 ug/ml | 1 ug/ml | 10 ug/ml |

**1. OVA**

**2. Micelle OVA-FITC**

medium

——— uptake 1h

·········· uptake 5h

EP 1 893 661 B1

FIG. 14B

1. OVA

2. OVA+CpG

3. Micelle OVA

4. Micelle OVA/CpG

isotype

medium

stimuli

CD80

CD86

FIG. 15

**PKN-U0126 CMFDA**
**concentration**

DC
total CD11c⁺

MQ
CD11c⁻ CD11b⁺

1 ug/ml

10 ug/ml

medium
uptake 5h

EP 1 893 661 B1

FIG. 16

FLT-3 spleen MACS sorted CD11c⁺
+
Micelle/plain OVA

Pulse 4h

Wash 3x

OT-1 splenocytes

OT-1 MACS sorted CD8+

5 days culture

Restimulation SIINFEKL + BFA
5 hours

ICC CD8⁺ IFNg⁺

# FIG. 17A

total OT-1 splenocytes

0.1 ug/ml    1 ug/ml

1. OVA protein

2. OVA protein +CpG

3. Micelle OVA protein

4. Micelle OVA protein/ CpG

5. Medium

total OT-1 splenocytes

| 1 | OVA |
| 2 | OVA+CpG |
| 3 | micelle OVA |
| 4 | micelle OVA/CpG |
| 5 | medium |

1 ug/ml
0.1 ug/ml

0  5  10 15 20 25 30 35 40 45 50

%CD8$^+$IFN$\gamma^+$/total CD8$^+$

# FIG. 17B

EP 1 893 661 B1

# FIG. 17C

**CD80**        **CD86**

|  | 4 h | 24 h | 4 h | 24 h |

1. OVA+CpG

2. Micelle OVA/CpG

isotype

untreated

stimuli

EP 1 893 661 B1

# FIG. 18A

EP 1 893 661 B1

# FIG. 18B

FIG. 18C

PRIME

1st BOOST

2nd BOOST

EP 1 893 661 B1

FIG. 18D

PRIME

1st BOOST

2nd BOOST

# FIG. 18E

**PRIME**

1. OVA
2. OVA+CpG
3. micelle OVA
4. micelle OVA:CpG
5. micelle OVA + U0126 PKN
6. micelle OVA:CpG + U0126 PKN

IgG total    IgG1    IgG 2a    IgG 2b

**BOOST 1**

1. OVA
2. OVA+CpG
3. micelle OVA
4. micelle OVA:CpG
5. micelle OVA + U0126 PKN
6. micelle OVA:CpG + U0126 PKN

IgG total    IgG 1    IgG 2a    IgG 2b

**BOOST 2**

1. OVA
2. OVA+CpG
3. micelle OVA
4. micelle OVA:CpG
5. micelle OVA + U0126 PKN
6. micelle OVA:CpG + U0126 PKN

IgG total    IgG 1    IgG 2a    IgG 2b

EP 1 893 661 B1

## FIG. 18F

1. OVA

2. OVA+CpG

3. micelle OVA

4. micelle OVA/CpG

5. micelle OVA + U0126 PKN

6. micelle OVA/CpG + U0126 PKN

# FIG. 19A

CMD     DMP     PCADK     CMD-PKNs     CMD     acetone

# FIG. 19B

### Hydrolysis of PCADK

— pH = 4.5
- ▲ - pH = 7.4

(1) Cyclohexane-dimethanol used in food packaging
(2) Acetone metabolic product of lipids
(3) PCADK Mw = 6,000, PD 1.5
(4) Yield 30-40%

EP 1 893 661 B1

FIG. 20

**Rhodhamine**

**Ebselen**

EP 1 893 661 B1

FIG. 21

EP 1 893 661 B1

# FIG. 22

Fastest hydrolysis hours-1day

Pentane diol-Polyketal

2nd fastest 1-2 days

PPADK

3rd fastest 2-3-weeks

PCADK

Slowest 6-8 weeks

Octane-diol Polyketal

EP 1 893 661 B1

# FIG. 23

| FITC-PKNs | Empty PKNs | IHC FITC-PKNs |

**Green dots are FITC-PKNs**
**Red stain is macrophage specific antibody**

## FIG. 24A

W/O Emulsion

Catalase (1.67mg/mL)  →  200µL H₂O

Polyketal (250.0mg/mL)  →  1.0mL CH₂Cl₂

Mixing
21,500rpm for 30s
Homogenization

1.2mL W/O Mixture

8% PVA (31-50kDa)
10.0mL H₂O

Mixing (at 4°C)
6,500rpm for 5min
Homogenization

W/O/W Emulsion

Increase Temperature Slowly up to 37°C

Add 30.0mL Water to W/O/W Mixture
(at 4°C)

Collection

Centrifuge 20,000xg for 20min

Vacuum Drying

Particles

# FIG. 24B

| SEM: 6000X magnification | SEM: 1000X magnification | Fluorescent Microscopy |
|---|---|---|

**Catalase encapsulation efficiency = 35%**

EP 1 893 661 B1

# FIG. 24C

**Hydrogen Peroxide can diffuse into particles
Intact catalase particles are active**

# FIG. 25

EP 1 893 661 B1

# FIG. 26

Acid
Hydrolysis

Low Molecular Weight
excretable compounds

Drug Loaded
Particle

Dynamic light scattering 100-300nm

# FIG. 27

● Nanoparticals formed by Solvent Evaporation (Single Emulsion)

| Batch | Polymer (mg) | PVA (mg) | $CH_3Cl$ (ul) | Buffer (ml) | Effective Diameter (nm) |
|---|---|---|---|---|---|
| 1 | 20 | 10 | 500 | 5 | 361.3 |
| 2 | 40 | 10 | 500 | 5 | 367.8 |
| 3 | 10 | 10 | 500 | 5 | 307.8 |
| 4 | 20 | 5 | 500 | 5 | 457.5 |
| 5 | 20 | 20 | 500 | 5 | 307.4 |
| 6 | 20 | 10 | 250 | 5 | 331.2 |
| 7 | 20 | 10 | 1000 | 5 | 378.2 |

● Conclusion: Particles formed are in 200-300nm range

EP 1 893 661 B1

REFERENCES CITED IN THE DESCRIPTION

**Patent documents cited in the description**

- WO 9323034 A

**Non-patent literature cited in the description**

- **PULENDRAN ; AHMED.** *Cell,* 2006, vol. 124, 849-863
- **PULENDRAN.** *Immunol. Rev.,* 2004, vol. 199, 227-250
- **PULENDRAN.** *J. Immunol.,* 2005, vol. 175, 2457-2465
- **AGRAWAL et al.** *J. Immunol.,* 2003, vol. 171, 4984-4989
- **DILLON et al.** *J. Clin. Immunol.,* 2006, vol. 116, 916-928
- **QUEREC et al.** *J. Exp. Med.,* 2006, vol. 203, 413-421
- **DILLON et al.** *J. clin. Immunol.,* 2006, vol. 116, 916-928
- **ANDERSON, J.M. et al.** *Adv. Drug Delivery Rev.,* 1997, vol. 28, 5-24
- **JAIN, R.A.** *Biomaterials,* 2000, vol. 21, 2475-2490
- **MATHIOWITZ, E. et al.** *J. Appl. Polym. Sci.,* 1988, vol. 35, 755-774
- **BERKLAND, C. et al.** *J. Controlled Release,* 2004, vol. 94, 129-141
- **STUBBS, M. et al.** *Mol. Med. Today,* 2000, vol. 6, 15-19
- **LEROUX, J.-C.** *Adv. Drug Delivery Rev.,* 2004, vol. 56, 925-926
- **HELLER, J. et al.** *Biomacromolecules,* 2004, vol. 5, 1625-1632
- **HELLER, J. et al.** *Adv Drug Delivery Rev.,* 2002, vol. 54, 1015-1039
- **BERRY, D. et al.** *Chem. Biol.,* 2004, vol. 11, 487-498
- **POTINENI, A. et al.** *J. Controlled Release,* 2003, vol. 86, 223-234
- **VAN ENDERT, PM.** *Biologicals,* 2001, vol. 29, 285-8
- **PURCELL, AW et al.** *Journal of Peptide Science,* 2003, vol. 9, 255-81
- **SHIRAI, M. et al.** *Journal of Virology,* 1994, vol. 68, 3334-42
- **HUNZIKER, IP et al.** *International Immunology,* 2002, vol. 14, 615-26
- **ERTL, HCJ et al.** *Vaccine,* 1996, vol. 14, 879-85
- **JACKSON, DC et al.** *Vaccine,* 1997, vol. 15, 1697-705
- **O. TAKEUCHI et al.** *Immunity,* 1999, vol. 11, 443-451
- **DILLON et al.** *J. Clin. Invest.,* 2006, vol. 116 (4), 916-28
- **A. OZINSKY et al.** *Proc. Natl. Acad. Sci. USA,* 2000, vol. 97, 13766-13771
- **K TAKEDA et al.** *Annu. Rev. Immunol.,* 2003, vol. 21, 335-376
- **BRAUN et al.** *J. Exp. Med,* 1999, vol. 189, 541-552
- **D'OSTIANI et al.** *J. Exp. Med.,* 2000, vol. 191, 1661-1674
- **PULENDRAN et al.** *J. Immunol.,* 2001, vol. 167, 5067-5076
- **TAKEUCHI et al.** *International Immunology,* 2001, vol. 13, 933-940
- **MACDONALD et al.** *J. Immunol.,* 2001, vol. 167, 1982-1988
- **BRAUN et al.** *J. Exp. Med.,* 1999, vol. 189, 541-552
- **ZUANY-AMORIM et al.** *Nature Reviews,* 2002, vol. 1, 797-807
- **TAKEDA et al.** *Ann. Rev. Immunol.,* 2003, vol. 21, 355-376
- **A-K YI et al.** *The Journal of Immunolgy,* 2002, vol. 168, 4711-4720
- **A. PUIG-KROGER et al.** *Blood,* 2001, vol. 98, 2175-2182
- **L.O. MURPHY et al.** *Nature Cell Biology,* 2002, vol. 4, 556-564
- **WONG G et al.** *Science,* 1985, vol. 228, 810
- **HORISBERGER M. A. et al.** *Journal of Virology,* March 1990, vol. 64 (3), 1171-81
- **LI YP et al.** *Journal of Immunology,* 01 February 1992, vol. 148 (3), 788-94
- **PIZARRO T. T. et al.** *Transplantation,* August 1993, vol. 56 (2), 399-404
- **BREVIARIO F. et al.** *Journal of Biological Chemistry,* 05 November 1992, vol. 267 (31), 22190-7
- **ESPINOZA-DELGADO I. et al.** *Journal of Immunology,* 01 November 1992, vol. 149 (9), 2961-8

- **ALGATE P. A. et al.** *Blood,* 01 May 1994, vol. 83 (9), 2459-68
- **CLUITMANS F. H. et al.** Annals of Hematology. June 1994, vol. 68, 293-8
- **MARTINEZ O. M. et al.** *Transplantation,* May 1993, vol. 55 (5), 1159-66
- **SHIMANE M. et al.** *Biochemical and Biophysical Research Communications,* 28 February 1994, vol. 199 (1), 26-32
- **KAY A. B. et al.** *Journal of Experimental Medicine,* 01 March 1991, vol. 173 (3), 775-8
- **DE WIT H et al.** *JOURNAL OF EXPERIMENTAL MEDICINE,* February 1994, vol. 86 (2), 259-64
- **SPRECHER E. et al.** *Archives of Virology,* 1992, vol. 126 (1-4), 253-69
- **HEFFERNAN MJ ; MURTHY N.** *Bioconjug. Chem.,* 2005, vol. 16 (6), 1340-2
- **JAIN RA.** *Biomaterials,* 2000, vol. 21 (23), 2475-90
- ADMET Polymerization. **WAGENER K. B. ; GOMEZ F. J.** Encyclopedia of Materials: Science and Technology. Elsevier, 2002, vol. 5, 48
- **O'REILLY et al.** *Chem. Mater.,* 2005, vol. 17 (24), 5976-5988
- **HANKER et al.** *Science,* 2005, vol. 309 (5738), 1200-05
- **LE, Z. et al.** *Langmuir,* 2005, vol. 21 (25), 11999-12006
- **FREIREICH, E. J et al.** *Cancer Chemother., Rep.,* 1966, vol. 50 (4), 219-244
- **AHSAN, F. ; RIVAS, 1. P. ; KHAN, M. A. ; TORRES-SUAREZ, A. I.** *J. Controlled Release,* 2002, vol. 79, 29-40
- **PRIOR, S. ; GANDER, B. ; BLARER, N. ; MERKLE, H. P. ; SUBIRÁ, M. L. ; IRACHE, J. M. ; GAMAZO, C.** *Eur. J. Pharm. Sci.,* 2002, vol. 15, 197-207
- **HAHN, S. K. ; JELACIC, S. ; MAIER, R. V. ; STAYTON, P. S. ; HOFFMAN, A. S.** *J. Biomater. Sci. Polymer Ed,* 2004, vol. 15, 1111-1119
- **WALTER, E. ; DREHER, D. ; KOK, M. ; THIELE, L. ; KIAMA, S. G. ; GEHR, P. ; MERKLE, H. P.** *J. Controlled Release,* 2001, vol. 76, 149-168
- **VAN APELDOORN, A. A. ; VAN MANEN, H.-J. ; BEZEMER, J. M. ; DE BRUIJN, J. D. ; VAN BLITTERSWIJK, C. A. ; OTTO, C.** *J. Am. Chem. Soc.,* 2004, vol. 126, 13226-13227
- **FU, K. ; PACK, D. W. ; KLIBANOV, A. M. ; LANGER, R.** *Pharm. Res.,* 2000, vol. 17, 100-106
- **SHENDEROVA, A. ; BURKE, T. G. ; SCHWENDEMAN, S. P.** *Pharm. Res.,* 1999, vol. 16, 241-248
- **FIFE, T. H. ; JAO, L. K.** *J. Org. Chem.,* 1965, vol. 30, 1492-1495
- **KWON, Y. J. ; STANDLEY, S. M. ; GOODWIN, A. P. ; GILLIES, E. R. ; FRÉCHET, J. M.** *J. Mol. Pharm.,* 2005, vol. 2, 83-91
- **MURTHY, N. ; CAMPBELL, J. ; FAUSTO, N. ; HOFFMAN, A. S. ; STAYTON, P. S.** *Bioconjugate Chem.,* 2003, vol. 14, 412-419
- **MURTHY, N. ; XU, M. ; SCHUCK, S. ; KUNISAWA, J. ; SHASTRI, N. ; FRÉCHET, J. M.** *J. Proc. Natl. Acad Sci. U.S.A.,* 2003, vol. 100, 4995-5000
- **STANDLEY, S. M. ; KWON, Y. J. ; MURTHY, N. ; KUNISAWA, J. ; SHASTRI, N. ; GUILLAUDEU, S. J. ; LAU, L. ; FRÉCHET, J. M.** *J. Bioconjugate Chem.,* 2004, vol. 15, 1281-1288
- **GILLIES, E. R. ; GOODWIN, A. P. ; FRÉCHET, J. M.** *J. Bioconjugate Chem.,* 2004, vol. 15, 1254-1263
- **LORETTE, N. B. ; HOWARD, W. L.** *J. Org. Chem.,* 1960, vol. 25, 521-525
- **PANYAM, J. ; WILLIAMS, D. ; DASH, A. ; LESLIE-PELECKY, D. ; LABHASETWAR, V.** *J. Pharm. Sci.,* 2004, vol. 93, 1804-1814